# FASCICULE DE BREVET EUROPEEN

(11) **EP 2 429 998 B1**
(45) Date de publication et mention de la délivrance du brevet: **27.11.2013**
(21) Numéro de dépôt: 10728740.1
(22) Date de dépôt: 11.05.2010
(51) Int. Cl.: C07D 231/12, C07D 261/10, C07D 277/22, C07D 401/14, C07D 413/14, A61K 31/439, A61P 25/00, A61P 29/00

(54) **DÉRIVÉS DE CYCLOPENTA[C]PYRROLYLALKYLCARBAMATES D'HÉTÉROCYCLES À 5 CHAÎNONS, LEUR PRÉPARATION ET LEUR APPLICATION EN THÉRAPEUTIQUE**
CYCLOPENTA[C]PYRROLYLALKYLCARBAMAT-DERIVATE AUS EINER 5-GLIEDRIGEN HETEROCYCLISCHEN VERBINDUNG, IHRE HERSTELLUNG UND THERAPEUTISCHE VERWENDUNG
5-MEMBERED HETEROCYCLIC COMPOUND CYCLOPENTA[C]PYRROLYLALKYLCARBAMATE DERIVATIVES, PREPARATION THEREOF, AND THERAPEUTIC USE THEREOF

(30) Priorité: 12.05.2009 FR 0902267
(43) Date de publication de la demande: 21.03.2012
(73) Titulaire: SANOFI, 75008 Paris (FR)
(72) Inventeur: ABOUABDELLAH, Ahmed, F-75013 Paris (FR); FAYOL, Aude, F-75013 Paris (FR); LOCHEAD, Alistair, F-75013 Paris (FR); SAADY, Mourad, F-75013 Paris (FR); VACHE, Julien, F-75013 Paris (FR); YAICHE, Philippe, F-75013 Paris (FR)
(74) Mandataire: Le Coupanec, Pascale A.M.P.
(86) Numéro de dépôt international: PCT/FR2010/050912
(87) Numéro de publication internationale: WO 2010/130943

(56) Documents cités:
- WO-A-2005/070910
- WO-A-2005/090322
- WO-A-2005/090347

## Description

L'invention a pour objet des dérivés de cyclopenta[c]pyrrolyl-alkylcarbamates d'hétérocycles à 5 chaînons, leur préparation et leur application en thérapeutique.

Il existe toujours une nécessité de trouver et de développer des produits inhibiteurs de l'enzyme FAAH (Fatty Acid Amide Hydrolase). Les composés de l'invention répondent à ce but.

Les composés de l'invention répondent à la formule générale (I) : 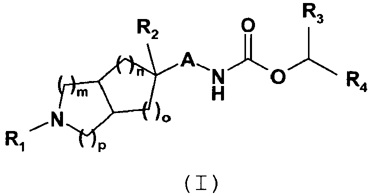 dans laquelle
R₂ représente un atome d'hydrogène, de fluor ou un groupe hydroxyle, cyano, trifluorométhyle, C₁₋₆-alkyle, C₁₋₆-alcoxy, NR₈R₉ ;
m et p ont pour valeur 1 ;
n et o ont la même valeur et ont pour valeur 0 ou 1 ;
A représente une liaison covalente ou un groupe C₁₋₈-alkylène ;
R₁ représente un groupe R₅ éventuellement substitué par un ou plusieurs groupes R₆ et/ou R₇ ;
R₅ représente un groupe choisi parmi un phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle ;
R₆ représente un atome d'halogène, un groupe cyano, - CH₂CN, nitro, hydroxyle, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène, C₃₋₇-cycloalkyle-C₁₋₃-alkylène-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ ou -O-(C₁₋₃-alkylène)-O- ;
R₇ représente un groupe choisi parmi un furanyle, pyrrolyle, thiènyle, oxazolyle, isoxazolyle, thiazolyle, isothiazolyle, imidazolyle, pyrazolyle, phényle, pyridinyle, pyridazinyle, pyrimidinyle, pyrazinyle, triazinyle; le ou les groupes R₇ pouvant être substitués par un ou plusieurs groupes R₆ identiques ou différents l'un de l'autre ;
R₃ représente un atome d'hydrogène, de fluor, un groupe C₁₋₆-alkyle ou un groupe trifluorométhyle ;
R₄ représente un hétérocycle à 5 chaînons choisi parmi un furanyle, pyrrolyle, thiènyle, thiazolyle, isothiazolyle, oxazolyle, isoxazolyle, pyrrazolyle, oxadiazolyle, thiadiazolyle, imidazole, triazolyle, tétrazolyle;
cet hétérocycle étant éventuellement substitué par un ou plusieurs substituants choisis parmi un atome d'halogène, un groupe C₁₋₆-alkyle, C₁₋₆-haloalkyle, C₃₋₇-cycloalkyle, C₃₋₇-cycloalkyle-C₁₋₃-alkylène, C₁₋₆-haloalcoxy, cyano, NR₈R₉, NR₈COR₉, NR₈CO₂R₉, NR₈SO₂R₉, NR₈SO₂NR₈R₉, COR₈, CO₂R₈, CONR₈R₉, CON (R₈) (C₁₋₃-alkylène-NR₁₀R₁₁), SO₂R₈, SO₂NR₈R₉, -O-(C₁₋₃-alkylène)-O- ;
R₈ et R₉ représentent indépendamment l'un de l'autre un atome d'hydrogène ou un groupe C₁₋₆-alkyle,
ou forment avec le ou les atomes qui les portent,
dans le cas de NR₈R₉, un cycle choisi parmi les cycles azétidine, pyrrolidine, pipéridine, morpholine, thiomorpholine, azépine, oxazépine ou pipérazine, ce cycle étant éventuellement substitué par un groupe C₁₋₆-alkyle ou benzyle ;
dans le cas de NR₈COR₉, un cycle lactame ; dans le cas de NR₈CO₂R₉, un cycle oxazolidinone, oxazinone ou oxazépinone; dans le cas de NR₈SO₂R₉, un cycle sultame ; dans le cas de NR₈SO₂NR₈R₉, un cycle dioxyde de thiazolidine ou dioxyde de thiadiazinane ;
R₁₀ et R₁₁ représentent indépendamment l'un de l'autre un atome d'hydrogène ou un groupe C₁₋₆-alkyle.

Parmi les composés de formule générale (I), un premier sous-groupe de composés est constitué des composés pour lesquels R₂ représente un atome d'hydrogène ou un atome de fluor. Parmi les composés de formule générale (I), un second sous-groupe de composés est constitué des composés pour lesquels R₂ représente un atome d'hydrogène. Parmi les composés de formule générale (I), un troisième sous-groupe de composés est constitué des composés pour lesquels R₂ représente un atome de fluor.

Parmi les composés de formule générale (I), un quatrième sous-groupe de composés est constitué des composés pour lesquels m, n, o et p ont pour valeur 1. Parmi les composés de formule générale (I), un cinquième sous-groupe est constitué des composés pour lesquels m et p ont pour valeur 1, et, n et o ont pour valeur 0.

Parmi les composés de formule générale (I), un sixième sous-groupe de composés est constitué des composés pour lesquels A représente une liaison covalente, un méthylène ou un éthylène.

Parmi les composés de formule générale (I), un septième sous-groupe de composés est constitué des composés pour lesquels A représente une liaison covalente.

Parmi les composés de formule générale (I), un huitième sous-groupe de composés est constitué des composés pour lesquels A représente un méthylène. Parmi les composés de formule générale (I), un neuvième sous-groupe de composés est constitué des composés pour lesquels A représente un éthylène.

Parmi les composés de formule générale (I), un dixième sous-groupe de composés est constitué des composés pour lesquels R₁ représente un groupe R₅ non substitué ou substitué par un ou plusieurs groupes R₆ et/ou R₇ ;
R₅ représente un groupe pyridinyle ou quinolinyle ;
R₆ représente un atome d'halogène, plus particulièrement un atome de fluor ou de brome, ou un groupe C₁₋₆-haloalkyle, plus particulièrement trifluorométhyle, ou un groupe C₁₋₆-alkyle, plus particulièrement un groupe méthyle;
R₇ représente un groupe choisi parmi pyrazolyle, pyridinyle et phényle, ces derniers pouvant être substitué par un ou plusieurs groupes R₆ identiques ou différents
l'un de l'autre.

Parmi les composés de formule générale (I), un onzième sous-groupe de composés est constitué des composés pour lesquels R₁ représente un groupe R₅ substitué par un ou plusieurs groupes R₆ et/ou R₇ ;
R₅ représente un groupe pyridinyle, plus particulièrement une pyridin-2-yle;
R₆ représente un atome d'halogène, plus particulièrement un atome de fluor ou de brome, ou un groupe C₁₋₆-haloalkyle, plus particulièrement trifluorométhyle ou un groupe C₁₋₆-alkyle, plus particulièrement un groupe méthyle;
R₇ représente un groupe choisi parmi pyrazolyle, pyridinyle et phényle, ces derniers pouvant être substitué par un ou plusieurs groupes R₆ identiques ou différents l'un de l'autre.

Parmi les composés de formule générale (I), un douzième sous-groupe de composés est constitué des composés pour lesquels R₁ représente un groupe R₅ substitué par un ou plusieurs groupes R₆;
R₅ représente un groupe quinolinyle, plus particulièrement une quinolin-2-yle;
R₆ représente un atome d'halogène, plus particulièrement un atome de fluor.

Parmi les composés de formule générale (I), un treizième sous-groupe de composés est constitué des composés pour lesquels R₃ représente un atome d'hydrogène.

Parmi les composés de formule générale (I), un quatorzième sous-groupe de composés est constitué des composés pour lesquels R₄ représente un groupe choisi parmi un thiazolyle, un triazolyle, ou un isoxazolyle;
ce groupe étant non substitué ou substitué par un ou plusieurs groupes C₁₋₆-alkyle, CO₂R₈ ou CONR₈R₉;
R₈ et R₉ représentent indépendamment l'un de l'autre un atome d'hydrogène ou un groupe C₁₋₆-alkyle. Plus particulièrement, le groupe C₁₋₆-alkyle est un méthyle ou éthyle.

Parmi les composés de formule générale (I), un quinzième sous-groupe de composés est constitué des composés pour lesquels R₄ représente un groupe thiazol-4-yle; ce groupe étant non substitué.

Parmi les composés de formule générale (I), un seizième sous-groupe de composés est constitué des composés pour lesquels R₄ représente un groupe isoxazol-5-yle; ce groupe étant substitué par un ou plusieurs groupes CO₂R₈ ou CONR₈R₉;

R₈ et R₉ représentent indépendamment l'un de l'autre un atome d'hydrogène ou un groupe C₁₋₆-alkyle. Plus particulièrement, le groupe C₁₋₆-alkyle est un méthyle ou éthyle.

Parmi les composés de formule générale (I), un dix-septième sous-groupe de composés est constitué des composés pour lesquels R₄ représente un groupe 1H-1,2,4-triazol-5-yle; ce groupe étant substitué par un ou plusieurs groupes C₁₋₆-alkyle.

Parmi les composés de formule générale (I), un dix-huitième sous-groupe de composés est constitué par les composés de formule générale (I) dans laquelle à la fois R₁ et/ou R₂ et/ou R₃ et/ou R₄ et/ou n et/ou m et/ou o et/ou p et/ou A sont tels que définis dans les groupes ci-dessus.

Parmi les composés de formule générale (I), les composés suivants peuvent être cités (nomenclature IUPAC générée par le logiciel AutoNom):
1. ({(3a*R*,5*s*,6a*S*)-2-[6-(trifluoromethyl)pyridin-2-yl]octahydrocyclopenta[*c*]pyrrol-5-yl}méthyl)carbamate de thiazol-4-ylméthyle (exo)
2. {[(3a*R*,5*s*,6a*S*)-2-(5-bromopyridin-2-yl)octahydrocyclopenta[*c*]pyrrol-5-yl]méthyl}carbamate de thiazol-4-ylméthyle (exo)
3. ({(3a*R*,5*s*,6a*S*)-2-[5-(4-fluorophenyl)pyridin-2-yl]octahydrocyclopenta[*c*]pyrrol-5-yl}méthyl)carbamate de thiazol-4-ylméthyle (exo)
4. ({(3a*R*,5*s*,6a*S*)-2-[5-(1-méthyl-1*H*-pyrazol-4-yl)pyridin-2-yl]octahydrocyclopenta[*c*]pyrrol-5-yl}méthyl)carbamate de thiazol-4-ylméthyle (exo)
5. {(3a*R*,5*s*,6a*S*)-2-[6-(trifluorométhyl)pyridin-2-yl]octahydrocyclopenta[*c*]pyrrol-5-yl}carbamate de thiazol-4-ylméthyle (exo)
6. [(3a*R*,5*s*,6a*S*)-2-(6-fluoroquinolin-2-yl)octahydrocyclopenta[*c*]pyrrol-5-yl]carbamate de thiazol-4-ylméthyle (exo)
7. ({(3a*R*,5*s*,6a*S*)-2-[5-(4-fluorophenyl)pyridin-2-yl]octahydrocyclopenta[*c*]pyrrol-5-yl}methyl)carbamate de (1-méthyl-1*H*-1,2,4-triazol-5-yl)méthyle (exo)
8. {[(3a*R*,5*s*,6a*S*)-2-(5'-fluoro-2,3'-bipyridin-6-yl)octahydrocyclopenta[*c*]pyrrol-5-yl]méthyl}carbamate de (1-méthyl-1*H*-1,2,4-triazol-5-yl)méthyle (exo)
9. ({(3a*R*,5*s*,6a*S*)-2-[5-(4-fluorophényl)pyridin-2-yl]octahydrocyclopenta[*c*]pyrrol-5-yl}méthyl)carbamate de (3-carbamoylisoxazol-5-yl)méthyle (exo)
10. {2-[(3a*R*,5*r*,6a*S*)-2-(5-bromopyridin-2-yl)octahydrocyclopenta[*c*]pyrrol-5-yl]éthyl}carbamate de [3- (méthylcarbamoyl)isoxazol-5-yl]méthyle (endo)
11. {2-[(3a*R*,5*r*,6a*S*)-2-(5-bromopyridin-2-yl)octahydrocyclopenta[*c*]pyrrol-5-yl]éthyl}carbamate de (3-carbamoylisoxazol-5-yl)méthyle (endo)
12. {[(3a*R*,5*r*,6a*S*)-5-fluoro-2-(6-fluoroquinolin-2-yl)octahydrocyclopenta[*c*]pyrrol-5-yl]méthyl}carbamate de (3-carbamoylisoxazol-5-yl)méthyle (exo)
13. {[(3a*R*,5*s*,6a*S*)-2-(6-fluoroquinolin-2-yl)octahydrocyclopenta[*c*]pyrrol-5-yl]méthyl}carbamate de [3-(méthylcarbamoyl)isoxazol-5-yl]méthyle (exo).
14. [3-(6-fluoroquinolin-2-yl)-3-azabicyclo[3.1.0]hex-6-yl]carbamate de [3-(méthylcarbamoyl)isoxazol-5-yl]méthyle
15. 5-{[({3-[5-(4-fluorophényl)pyridin-2-yl]-3-azabicyclo[3.1.0]hex-6-yl}carbamoyl)oxy]méthyl}isoxazole-3- carboxylate d'éthyle
16. [3-(6-fluoroquinolin-2-yl)-3-azabicyclo[3.1.0]hex-6-yl]carbamate de (3-carbamoylisoxazol-5-yl)méthyle
17. {3-[5-(4-fluorophényl)pyridin-2-yl]-3-azabicyclo[3.1.0]hex-6-yl}carbamate de (3-carbamoylisoxazol-5-yl)méthyle
18. {3-[5-(4-fluorophényl)pyridin-2-yl]-3-azabicyclo[3.1.0]hex-6-yl}carbamate de [3-(méthylcarbamoyl)isoxazol-5-yl]méthyle
19. {3-[5-(4-fluorophényl)pyridin-2-yl]-3-azabicyclo[3.1.0]hex-6-yl}carbamate de [3-(diméthylcarbamoyl)isoxazol-5-yl]méthyle

Les composés de formule générale (I) peuvent comporter un ou plusieurs carbones asymétriques. Ils peuvent exister sous forme d'énantiomères ou de diastéréoisomères. Les composés de formule générale (I) peuvent également exister sous forme de stéréoisomères *cis* ou *trans.* Ces stéréoisomères, énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Dans le cadre de l'invention, on entend par :
- C_{t-z} où t et z peuvent prendre les valeurs de 1 à 8, une chaîne carbonée pouvant avoir de t à z atomes de carbone, par exemple C₁₋₃ une chaîne carbonée qui peut avoir de 1 à 3 atomes de carbone ;
- alkyle, un groupe aliphatique saturé, linéaire ou ramifié; par exemple un groupe C₁₋₆-alkyle représente une chaîne carbonée de 1 à 6 atomes de carbone, linéaire ou ramifiée, plus particulièrement un méthyle, éthyle, propyle, isopropyle, butyle, isobutyle, *tert*-butyle, pentyle, hexyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, plus particulièrement un cyclopropyle, cyclobutyle, cyclopentyle, cyclohexyle, cycloheptyle ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- haloalkyle, un groupe alkyle dont un ou plusieurs atomes d'hydrogène ont été substitués par un atome d'halogène ;
- haloalcoxy, un groupe alcoxy dont un ou plusieurs atomes d'hydrogène ont été substitués par un atome d'halogène ;
- halothioalkyle, un groupe thioalkyle dont un ou plusieurs atomes d'hydrogène ont été substitués par un atome d'halogène ;
- atome d'halogène, un fluor, un chlore, un brome ou un iode.
- Le terme '*exo*' correspond au groupement -R2 en trans par rapport aux hydrogènes de jonction de cycle. Le terme '*endo*' correspond au groupement -R2 en cis par rapport aux hydrogènes de jonction de cycle.
- r et s indique la stéréochimie des atomes de carbones pseudo-asymétriques, selon les règles IUPAC.

Les composés de l'invention peuvent être préparés selon différentes méthodes, illustrées par les schémas qui suivent. Ces méthodes, ainsi que les composés intermédiaires utilisés, sont un objet de la présente invention.

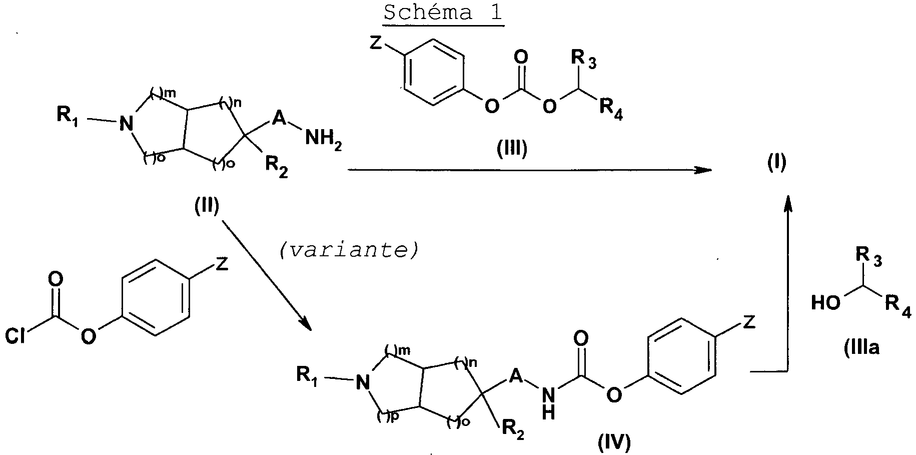

Ainsi, une première méthode (schéma 1) consiste à faire réagir une amine de formule générale (II), dans laquelle A, R₁, R₂, m, n, o et p sont tels que définis dans la formule générale (I) définie ci-dessus, avec un carbonate de formule générale (III) dans laquelle Z représente un atome d'hydrogène ou un groupe nitro, R₃ et R₄ sont tels que définis dans la formule générale (I) définie ci-dessus, en présence d'une base telle que la triéthylamine, la pyridine, la *N,N*-diméthylaminopyridine ou la diisopropyléthylamine, dans un solvant tel que le toluène ou le dichloroéthane, à une température comprise entre la température ambiante et la température de reflux du solvant.

Une variante d'obtention des composés de formule générale (I) (schéma 1) consiste à faire réagir une amine de formule générale (II), telle que définie ci-dessus, avec le chloroformiate de phényle ou de 4-nitro-phényle, en présence d'une base telle que la triéthylamine ou la diisopropyléthylamine, dans un solvant tel que le dichlorométhane ou le tétrahydrofurane, à une température comprise entre 0°C et la température ambiante, pour conduire au dérivé carbamate de formule générale (IV), dans laquelle A, R₁, R₂, m, n, o et p sont tels que définis dans la formule générale (I) définie ci-dessus, et Z représente un atome d'hydrogène ou un groupe nitro. Le dérivé carbamate de formule générale (IV) ainsi obtenu est ensuite transformé en composé de formule générale (I), par action d'un alcool de formule générale HOCHR₃R₄ (IIIa), telle que définie ci-dessus, en présence d'une base telle que la triéthylamine, la pyridine, la *N,N*-diméthylaminopyridine ou la diisopropyléthylamine, dans un solvant tel que le toluène ou le dichloroéthane, à une température comprise entre la température ambiante et la température de reflux du solvant.

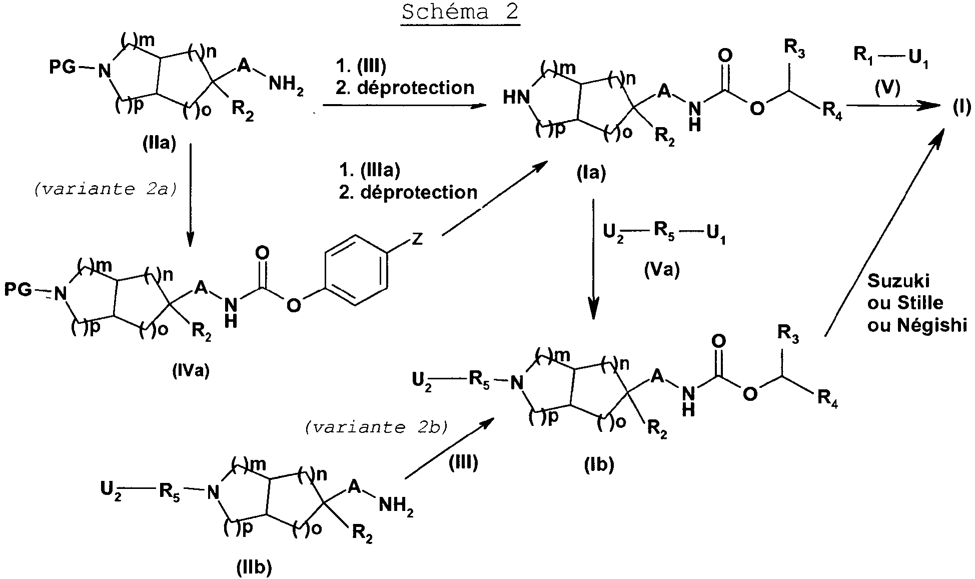

Une seconde méthode (schéma 2) consiste à faire réagir dans un premier temps une amine de formule générale (IIa), dans laquelle A, R₂, m, n, o et p sont tels que définis dans la formule générale (I) définie ci-dessus, et PG représente un groupe protecteur tel qu'un Boc (*tert*-butyloxycarbonyl), un Cbz (benzyloxycarbonyl), un benzyle ou un benzhydrile, avec un carbonate de formule générale (III) telle que définie ci-dessus, dans les conditions décrites ci-dessus lors de la réaction de l'amine de formule générale (II) avec le carbonate de formule générale (III), suivie d'une réaction de déprotection, par exemple en présence d'une solution d'acide chlorhydrique (5N) dans l'isopropanol ou le dioxane, pour obtenir l'intermédiaire de formule générale (Ia), dans laquelle A, R₂, R₃, R₄, m, n, o et p sont tels que définis dans la formule générale (I).

Une variante d'obtention des intermédiaires de formule générale (Ia) (schéma 2, variante 2a) consiste à faire réagir une amine de formule générale (IIa), telle que définie ci-dessus, avec le chloroformiate de phényle ou de 4-nitro-phényle, en présence d'une base telle que la triéthylamine ou la diisopropyléthylamine, dans un solvant tel que le dichlorométhane ou le tétrahydrofurane, à une température comprise entre 0°C et la température ambiante, pour conduire au dérivé carbamate de formule générale (IVa), dans laquelle A, R₂, m, n, o et p sont tels que définis dans la formule générale (I) définie ci-dessus, PG est tel que défini ci-dessus et Z représente un atome d'hydrogène ou un groupe nitro. Le dérivé carbamate de formule générale (IVa) ainsi obtenu est ensuite transformé en composé de formule générale (Ia), par action d'un alcool de formule générale HOCHR₃R₄ (IIIa), telle que définie ci-dessus, en présence d'une base telle que la triéthylamine, la pyridine, la *N,N-*diméthylaminopyridine ou la diisopropyléthylamine, dans un solvant tel que le toluène ou le dichloroéthane, à une température comprise entre la température ambiante et la température de reflux du solvant, suivie par une réaction de déprotection, par exemple en présence d'une solution d'acide chlorhydrique (5N) dans l'isopropanol ou le dioxane.

Le composé de formule générale (I) est ensuite obtenu par réaction du composé de formule générale (Ia) avec un dérivé de formule générale (V), dans laquelle R₁ est tel que défini dans la formule générale (I) et U₁ représente un atome d'halogène ou un groupe triflate, en utilisant les conditions de réactions de substitution nucléophiles aromatiques ou hétéroaromatiques, par exemple au moyen d'une base telle que la triéthylamine, la diisopropyléthylamine, la pyridine ou la *N,N*-diméthylaminopyridine dans un solvant tel que le dichlorométhane, le dichloroéthane, l'acétonitrile, la *N,N*-diméthylformamide, le dioxane ou le tétrahydrofurane, à une température comprise entre 0°C et la température de reflux du solvant. Cette transformation peut être également réalisée en utilisant les conditions de N-arylation ou *N*-hétéroarylation de Buchwald, par exemple au moyen d'un catalyseur au Palladium ou au Cuivre.

Selon le schéma 2, les composés de formule générale (I), dans laquelle R₁ représente un groupe R₅ substitué notamment par un groupe R₆ de type C₁₋₆-alkyle, C₃₋₇-cycloalkyle ou C₃₋₇-cycloalkyle-C₁₋₃-alkylène, ou par un groupe R₇ tel que défini dans la formule générale (I) définie ci-dessus, peuvent être également préparés selon une réaction de couplage, catalysée au moyen d'un métal de transition, par exemple le Palladium (0), réalisée sur le composé de formule générale (Ib), dans laquelle A, R₂, R₃, R₄, R₅, m, n, o et p sont tels que définis dans la formule générale (I) et U₂ représente un atome de chlore, de brome, d'iode ou un groupement triflate, U₂ étant dans la position où l'on souhaite introduire le groupe R₆ ou R₇ (schéma 2):
soit par une réaction de type Suzuki, par exemple au moyen d'un acide boronique d'alkyle, de cycloalkyle, d'aryle ou d'hétéroaryle,
soit selon une réaction de type Stille, par exemple en utilisant un dérivé *tri*-alkylstanneux d'aryle ou d'hétéroaryle
soit par une réaction de type Négishi, par exemple en utilisant un dérivé zincate d'halogénure d'alkyle, de cycloalkyle, d'aryle ou d'hétéroaryle.

L'intermédiaire de formule générale (Ib) telle que définie ci-dessus est préalablement obtenu en faisant réagir une amine de formule générale (Ia) telle que définie ci-dessus avec un dérivé de formule générale (Va) dans laquelle R₅, U₁ et U₂ sont tels que définis ci-dessus en utilisant les réactions de substitution nucléophiles aromatiques, hétéroaromatiques ou de *N*-arylation, *N*-hétéroarylation de Buchwald, par exemple au moyen d'un catalyseur au Palladium ou au Cuivre.

Une variante d'obtention des intermédiaires de formule générale (Ib) (schéma 2, variante 2b) consiste à faire réagir dans un premier temps une amine de formule générale (IIb), dans laquelle A, R₅, R₂, m, n, o et p sont tels que définis dans la formule générale (I) définie ci-dessus, et U₂ est tel que défini ci-dessus, avec un carbonate de formule générale (III) telle que définie ci-dessus, dans les conditions décrites ci-dessus lors de la réaction de l'amine de formule générale (II) avec le carbonate de formule générale (III), pour obtenir l'intermédiaire de formule générale (Ib), dans laquelle A, R₅, R₂, R₃, R₄, m, n, o et p sont tels que définis dans la formule générale (I), et U₂ est tel que défini ci-dessus.

Un autre objet de la présente invention se rapporte aux composés de formule générale (Ia), 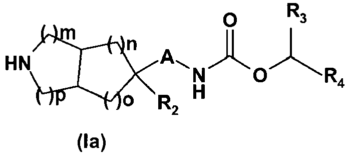 dans laquelle A, R₂, R₃, R₄, m, n, o et p sont tels que définis dans la formule générale (I). parmi ces composés, on peut citer :
[(3a*R*,5*s*,6a*S*)-octahydrocyclopenta[*c*]pyrrol-5-ylméthyl]carbamate de thiazol-4-ylméthyle ;
(3a*R*,5*s*,6a*S*)-octahydrocyclopenta[*c*]pyrrol-5-ylcarbamate de thiazol-4-ylméthyle ;
[(3a*R*,5*s*,6a*S*)-octahydrocyclopenta[*c*]pyrrol-5-ylméthyl]carbamate de (1-méthyl-1*H*-1,2,4-triazol-5-yl)méthyle ;
[(3a*R*,5*s*,6a*S*)-octahydrocyclopenta[*c*]pyrrol-5-ylméthyl] carbamate de (3-carbamoylisoxazol-5-yl)méthyle ;
5-{[({2-[(3a*R*,5*r*,6a*S*)-octahydrocyclopenta[*c*]pyrrol-5-yl]éthyl}carbamoyl)oxy]méthyl}isoxazole-3-carboxylate d'éthyle ;
5-{[({[(3a*R*,5*r*,6a*S*)-5-fluorooctahydrocyclopenta[*c*]pyrrol-5-yl]méthyl}carbamoyl)oxy]méthyl}isoxazole-3-carboxylate d'éthyle;
5-{[(3-azabicyclo[3.1.0]hex-6-ylcarbamoyl)oxy]methyl}isoxazole-3-carboxylate d'éthyle.

Un autre objet de la présente invention se rapporte aux composés de formule générale (II), 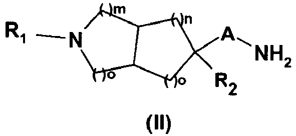 dans laquelle A, R₁, R₂, m, n, o et p sont tels que définis dans la formule générale (I).

Un autre objet de la présente invention se rapporte aux composés de formule générale (IV), 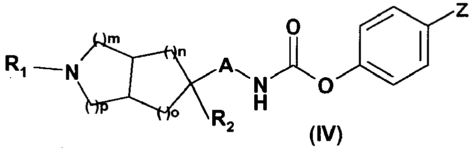 dans laquelle A, R₁, R₂, m, n, o et p sont tels que définis

dans la formule générale (I), et Z représente un atome d'hydrogène ou un groupe nitro.

Les autres composés de formules générales (II), (IIa), (IIb), (III), (IIIa), (V) et (Va) ainsi que les autres réactifs sont disponibles dans le commerce ou décrits dans la littérature, ou bien peuvent être préparés selon des méthodes qui y sont décrites ou qui sont connues de l'homme du métier.

Les exemples qui suivent illustrent la préparation de quelques composés de l'invention. Ces exemples ne sont pas limitatifs et ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus.

PF(°C) représente le point de fusion en degrés Celsius.

R_{f} indique le temps de rétention obtenu par analyse CCM (Chromatographie sur Couche Mince).

Les numéros indiqués entre parenthèses dans les titres des exemples correspondent à ceux de la 1ère colonne des tableaux ci-après.

La nomenclature UICPA (Union Internationale de Chimie Pure et Appliquée - IUPAC en anglais) a été utilisée pour la dénomination des composés dans les exemples ci-dessous.

### Exemple 1 (Composé N°2)

### {[(3aR,5s,6aS)-2-(5-bromopyridin-2-yl)octahydrocyclopenta[c]pyrrol-5-yl]méthyl} carbamate de thiazol-4-ylméthyle (exo)

### 1.1. (3aR,5s,6aS)-5-cyanohexahydrocyclopenta[c]pyrrole-2(1H)-carboxylate de tert-butyle

2,3g (7,53 mmoles) de (3a*R*,5*r*,6a*S*)-5-[(méthylsulfonyl)oxy]hexahydrocyclopenta[*c*]pyrrole-2(1*H*)-carboxylate de *tert*-butyle (W02006108059) sont mis en solution dans 25 mL de diméthylsulfoxide puis 3,69 g (75,31 mmoles) de cyanure de sodium sont ajoutés. Le mélange est agité à 80°C pendant 1 heure.

On laisse revenir à température ambiante, on dilue par ajout d'eau et d'acétate d'éthyle. On extrait la phase aqueuse avec de l'acétate d'éthyle puis on sèche les phases organiques réunies sur du sulfate de sodium, on filtre puis on évapore le filtrat à sec. On purifie le résidu ainsi obtenu par chromatographie sur gel de silice avec comme éluant un mélange 95/5 à 85/15 de cyclohexane et d'acétate d'éthyle. On obtient 0,81 g de produit sous forme d'un solide blanc.

Point de fusion (°C) : 62-64°C

RMN-¹H (DMSO) δ (ppm) : 3,48 (m, 2H), 3,19 (m, 1H), 3,00 (m, 2H), 2,78 (m, 2H), 1,99 (m, 2H), 1,88 (m, 2H), 1,39 (s, 9H).

### 1.2. (3aR,5s,6aS)-5-(aminométhyl)hexahydrocyclopenta[c]pyrrole-2(1H)-carboxylate de tert-butyle

0,54 g (2, 29 mmoles) de (3a*R*,5*s*,6a*S*)-5-cyanohexahydrocyclopenta[*c*]pyrrole-2(1*H*)-carboxylate de *tert*-butyle, obtenu à l'étape 1.1., est mis en solution dans 20 mL d'une solution de soude à 1M dans l'éthanol en présence d'une quantité catalytique de Nikel de Raney (50% dans l'eau). La solution est agitée à température ambiante sous une pression d'hydrogène de 4 bars à l'aide d'un appareil de Parr pendant 6 heures. Après évaporation de l'éthanol, la solution est reprise dans de l'eau et du dichlorométhane. La phase aqueuse est extraite plusieurs fois avec du dichlorométhane. Les phases organiques réunies sont ensuite lavées avec une solution aqueuse saturée en chlorure de sodium puis séchées sur sulfate de sodium. Après filtration et évaporation à sec, on obtient 0,550g d'une huile incolore.

LC-MS : M+H = 241

RMN-¹H (DMSO) δ (ppm) : 3,45 (m, 2H), 2,98 (m, 2H), 2,64 (m, 2H), 2,45 (d, 2H), 2,03 (m, 1H), 1,54 (m, 2H), 1,44 (m, 2H), 1,40 (s, 9H).

### 1.3. (3aR,5s,6aS)-5-({[(1,3-thiazol-4-ylméthoxy)carbonyl]amino}méthyl)hexahydrocyclopenta[c]pyrrol e-2(1H)-carboxylate de tert-butyle

0,550 g (2,29 mmoles) de (3a*R*,5*s*,6a*S*)-5-(aminométhyl)hexahydrocyclopenta[*c*]pyrrole-2(1*H*)-carboxylate de *tert*-butyle, obtenu à l'étape 1.2., 0,028 g (0,23 mmole) de *N,N*-diméthyl-4-aminopyridine, 1,20 mL (6,87 mmoles) de *N,N*-diisopropyléthylamine et 0,705 g (2,52 mmoles) de 4-nitro-phényl-carbonate de thiazol-4-ylméthyle (WO2008013834) sont mis en solution dans 20 mL de 1,2-dichloroéthane. Le mélange est agité à 70°C pendant 1 heure et 30 minutes. Après retour à température ambiante, de l'eau est ajoutée au milieu réactionnel. Après extraction de la phase aqueuse avec du dichlorométhane, les phases organiques sont successivement lavées trois fois par une solution aqueuse de soude 1M puis 2 fois par une solution aqueuse saturée en chlorure d'ammonium. Après avoir séché les phases organiques sur sulfate de sodium, on filtre et on évaporer le filtrat à sec. On obtient ainsi 1 g de produit brut engagé tel quel dans l'étape suivante.

### 1.4.[(3aR,5s,6aS)-octahydrocyclopenta[c]pyrrol-5-ylméthyl]carbamate de thiazol-4-ylméthyle.

0,905g (2,29 mmoles) de (3a*R*,5*s*,6a*S*)-5-({[(thiazol-4-ylméthoxy)carbonyl]amino}méthyl)hexahydrocyclopenta[*c*]pyrrol e-2(1*H*)-carboxylate de *tert*-butyle, obtenu à l'étape 1.3., est mis en solution dans 20 mL de dichlorométhane puis 1,93 mL (22,90 mmoles) d'acide trifluoroacétique sont ajoutés. Le mélange est agité à température ambiante pendant 3 heures.

Un traitement avec de la soude 1M après extraction au dichlorométhane puis séchage sur sulfate de sodium et évaporation à sec, conduit à 0,66g d'une huile orange.

LC-MS : M+H = 282

RMN-¹H (DMSO) δ (ppm) : 8, 95 (s, 1H), 7,48 (s, 1H), 7,10 (large s, 1H), 4,92 (s, 2H), 2,80 (m, 4H), 2,33 (m, 2H), 2,20 (m, 2H), 1,98 (m, 1H), 1,23 (m, 4H).

### 1.5. {[(3aR,5s,6aS)-2-(5-bromopyridin-2-yl)octahydrocyclopenta[c]pyrrol-5-yl]méthyl}carbamate thiazol-4-ylméthyle

0,40 g (1,42 mmole) de (3a*R*,5*s*,6a*S*) - octahydrocyclopenta[*c*]pyrrol-5-ylméthyl]carbamate thiazol-4-ylméthyle, obtenu à l'étape 1.4., est mis en solution dans 4 mL d'acétonitrile dans un tube scellé. 0,27 g (1,56 mmole) de 5-bromo-2-fluoro-pyridine et 0,5 mL de *N,N-*diisopropyléthylamine sont ajoutés. Le mélange est agité à 100°C pendant 6 heures.

Après retour à température ambiante, on ajoute de l'eau et de l'acétate d'éthyle. La phase aqueuse est extraite plusieurs fois avec de l'acétate d'éthyle. Les phases organiques réunies sont lavées avec une solution aqueuse saturée en chlorure d'ammonium puis séchées sur sulfate de sodium. Une purification sur colonne de gel de silice en éluant avec un mélange 99/1 puis 96/4 de dichlorométhane et de méthanol a permis d'obtenir 0,30 g de produit sous forme d'un solide beige.

Point de fusion (°C) : 114-116°C

LC-MS : M+H = 437

RMN-¹H (DMSO) δ (ppm) : 9,10 (s, 1H), 8,12 (s, 1H), 7,65 (m, 2H), 7,30 (large s, 1H), 6,48 (d, 1H), 5,12 (s, 2H), 3,53 (m, 2H), 3,15 (m, 2H), 2,96 (m, 2H), 2,82 (m, 2H), 2,21 (m, 1H), 1,58 (m, 4H).

### Exemple 2 (Composé N°3)

### ({(3aR,5s,6aS)-2-[5-(4-fluorophényl)pyridin-2-yl]octahydrocyclopenta[c]pyrrol-5-yl}méthyl)carbamate de thiazol-4-ylméthyle (exo)

Sous atmosphère inerte, on introduit 0,125 g (0,29 mmole) de {[(3a*R*,5*s*,6a*S*)-2-(5-bromopyridin-2-yl)-octahydrocyclopenta[*c*]pyrrol-5-yl]méthyl}carbamate de thiazol-4-ylméthyle, préparé dans l'exemple 1, étape 1.5, 0,048 g (0,34 mmole) d'acide 4-fluorophénylboronique, 0,279 g (0,86 mmole) de carbonate de césium en suspension dans 2,5 ml d'un mélange 9/1 de tétrahydrofurane et d'eau. On ajoute ensuite 0,023 g (0,03 mmole) de PdCl₂dppf.CH₂Cl₂. On chauffe ensuite à environ 75°C pendant 2 heures et 30 minutes.

On laisse revenir à température ambiante, puis on reprend le filtrat avec de l'acétate d'éthyle et de l'eau, on sépare la phase aqueuse, on l'extrait deux fois avec de l'acétate d'éthyle, on lave les phases organiques réunies avec une solution aqueuse saturée en chlorure de sodium et on les sèche sur sulfate de sodium. Après évaporation du solvant, on purifie le résidu obtenu par chromatographie sur plaques préparatives en éluant avec de l'acétate d'éthyle. On obtient 0,07g d'un solide beige.

Point de fusion (°C) : 129-131°C

LC-MS : M+H = 453

RMN-¹H (DMSO) δ (ppm) : 9,10 (s, 1H), 8,38 (s, 1H), 7,80 (d, 1H), 7,61 (m, 3H), 7,32 (large s, 1H), 7,21 (m, 2H), 6,58 (d, 1H), 5,11 (s, 2H), 3,60 (m, 2H), 3,12 (m, 2H), 2,99 (m, 2H), 2,81 (m, 2H), 2,21 (m, 1H), 1,56 (m, 4H).

### Exemple 3 (Composé N°5)

### {(3aR,5s,6aS)-2-[6-(trifluorométhyl)pyridin-2-yl]octahydrocyclopenta[c]pyrrol-5-yl}carbamate de thiazol-4-ylméthyle (exo)

### 3.1. (3aR,5s,6aS)-5-aminohexahydrocyclopenta[c]pyrrole-2(1H)-carboxylate de tert-butyle

1,0 g (3,27 mmoles) de (3a*R*,5*r*,6a*S*)-5-[(méthylsulfonyl)oxy]hexahydrocyclopenta[*c*]pyrrole-2(1*H*)-carboxylate de *tert*-butyle (WO2006108059) sont mis en solution dans 25 mL de *N,N*-diméthylformamide puis 0,29 g (4,46 mmoles) d'azoture de sodium sont ajoutés. Le mélange est ensuite agité à 90°C pendant 2 heures et 30 minutes.

On laisse revenir à température ambiante, on dilue par ajout d'eau et d'acétate d'éthyle. On extrait la phase aqueuse avec de l'acétate d'éthyle puis on sèche les phases organiques réunies sur sulfate de sodium, on filtre et on évapore le filtrat à sec. Le résidu obtenu est engagé tel quel dans l'étape qui suit.

Le composé (3a*R*,5*s*,6a*S*)-5-azidohexahydrocyclopenta[*c*]pyrrole-2(1*H*)-carboxylate de *tert*-butyle, obtenu précédemment, est mis en solution dans 15 mL d'éthanol avec 0,34 g (1,63 mmole) de catalyseur de Lindlar dans un appareil de Parr sous une pression de 20 psi d'hydrogène à température ambiante pendant 3 heures. Après filtration sur célite et évaporation à sec, on obtient (3*aR*,5*s*,6a*S*)-5-aminohexahydrocyclopenta[*c*]pyrrole-2(1*H*)-carboxylate de *tert*-butyle sous forme d'huile utilisé tel quel dans l'étape suivante.

LC-MS : M+H = 227

RMN-¹H (DMSO) δ (ppm) : 3,41 (m, 3H), 2,99 (m, 2H), 2,68 (m, 2H), 1,54 (m, 2H), 1,50 (m, 2H), 1,45 (s, 9H).

### 3.2. (3aR,5s,6aS)-octahydrocyclopenta[c]pyrrol-5-ylcarbamate de thiazol-4-ylméthyle

On procède suivant le mode opératoire décrit dans l'exemple 1, étape 1.3. A partir de 0,74 g (3,27 mmoles) de (3a*R*,5*s*,6a*S*)-5-aminohexahydrocyclopenta[*c*]pyrrole-2(1*H*)-carboxylate de *tert*-butyle, obtenu à l'étape 3.1., de 1,71 mL (9,81 mmoles) de *N,N*-diisopropyléthylamine, de 0,04 g (0,33 mmole) de *N,N*-diméthylaminopyridine, de 0,91 g (3,27 mmoles) de 4-nitro-phényl-carbonate de thiazol-4-ylméthyle (W02008013834), on obtient 1,5 g de produit brut engagé tel quel dans l'étape qui suit.

Le produit (3a*R*,5*s*,6a*S*)-5-{[(thiazol-4-ylméthoxy)carbonyl]amino}hexahydrocyclopenta[*c*]pyrrole-2(1*H*)-carboxylate de *tert*-butyle, obtenu précédemment, est mis en solution dans 30 mL de dichlorométhane puis 2,76 mL d'acide trifluoroacétique sont ajoutés. Le mélange est agité à température ambiante pendant 15 heures.

Après un traitement basique avec une solution aqueuse de soude 1M et extraction au dichlorométhane, on obtient 0,690 g de (3a*R*,5*s*,6a*S*)-octahydrocyclopenta[c]pyrrol-5-ylcarbamate de thiazol-4-ylméthyle sous forme d'un solide blanc.

Point de fusion (°C) : 135-137°C

LC-MS : M+H = 268

RMN-¹H (DMSO) δ (ppm): 9,14 (s, 1H), 7,70 (s, 1H), 7,25 (large s, 1H), 5,14 (s, 2H), 3,95 (m, 1H), 2,95 (m, 2H), 2,55 (m, 2H), 2,48 (m, 2H), 1,60 (m, 4H).

### 3.3. {(3aR,5s,6aS)-2-[6-(trifluorométhyl)pyridin-2-yl]octahydrocyclopenta[c]pyrrol-5-yl} carbamate de thiazol-4-ylméthyle

On procède suivant le mode opératoire décrit dans l'exemple 1, étape 1.5.. A partir de 0,1 g (0,37 mmole) de (3a*R*,5*s*,6a*S*)-octahydrocyclopenta[c]*p*y*r*rol-5-ylcarbamate de thiazol-4-ylméthyle, de 0,2 mL (1,12 mmole) de *N,N-*diisopropyléthylamine et de 0,07 g (0,45 mmole) de 2-fluoro-6-trifluorométhyl-pyridine, et après chromatographie sur plaque préparative de gel de silice en éluant avec de l'acétate d'éthyle, on obtient 0,08 g de produit pur sous forme d'un solide.

Point de fusion (°C) : 148-150°C

LC-MS : M+H = 413

RMN-¹H (DMSO) δ (ppm) : 9,11 (s, 1H), 7,70 (m, 2H), 7,40 (m, 1H), 7,01 (d, 1H), 6,78 (d, 1H), 5,12 (s, 2H), 4,00 (m, 1H), 3,58 (m, 2H), 3,28 (m, 2H), 2,90 (m, 2H), 1,78 (m, 4H).

### Exemple 4 (Composé N°8)

### {[(3aR,5s,6aS)-2-(5'-fluoro-2,3'-bipyridin-6-yl)octahydrocyclopenta[c]pyrrol-5-yl]méthyl}carbamate de (1-méthyl-1H-1,2,4-triazol-5-yl)méthyle (exo)

### 4.1 (3aR, 5s, 6aS) -5- ({[(4-nitrophénoxy)carbonyl]amino}méthyl)hexahydrocyclopenta[c]pyr role-2(1H)-carboxylate de tert-butyle

A une solution de 1,24 g (5,16 mmoles) de (3a*R*,5*s*,6a*S*)-5-(aminométhyl)hexahydrocyclopenta[c]pyrrole-2(1*H*)-carboxylate de *tert*-butyle, obtenu dans l'exemple 1, étape 1.2., et de 1,80 mL (10,32 mmoles) de *N,N*-diisopropyléthylamine dans 30 mL de 1,2-dichloroéthane, on ajoute une solution de 1,14 g (5,68 mmoles) de chloroformiate de *para*-nitrophényle dans 20 mL de 1,2-dichloroéthane à environ 0°C. Le mélange est ensuite agité à température ambiante pendant 15 heures.

On ajoute au milieu réactionnel une solution aqueuse saturée de chlorure d'ammonium. La phase aqueuse est extraite plusieurs fois au dichlorométhane. Les phases organiques sont ensuite lavées avec une solution aqueuse saturée de chlorure d'ammonium puis séchées sur sulfate de sodium. Une purification sur colonne de gel de silice en éluant avec un mélange 99/1 puis 90/10 de cyclohexane et d'acétate d'éthyle a permis d'obtenir 1,30 g de produit sous forme d'un solide.

Point de fusion (°C) : 132-134°C

RMN-¹H (DMSO) δ (ppm) : 8,26 (d, 2H), 8,06 (m, 1H), 7,40 (d, 2H), 3,44 (m, 2H), 3,01 (m, 4H), 2,67 (m, 2H), 2,25 (m, 1H), 1,58 (m, 4H), 1,38 (s, 9H).

### 4.2. 6-Bromo-5'-fluoro-[2,3']bipyridinyle

A une solution de 2,00 g (8,44 mmoles) de 2,6-dibromo-pyridine dans 80 mL d'un mélange 4/1 de toluène et d'éthanol, on ajoute 1,18 g (8,44 mmoles) d'acide 5-fluoropyridine-3-boronique, 0,48 g (0,42 mmole) de Pd(PPh₃)₄ et 25 mL d'une solution aqueuse 1M de carbonate de sodium. Le mélange et agité à 90 °C pendant 2 heures.

On laisse revenir à température ambiante, on extrait plusieurs fois la phase aqueuse avec de l'acétate d'éthyle. On sèche les phases organiques réunies sur sulfate de sodium, on filtre et on évapore à sec. On purifie le résidu ainsi obtenu sur colonne de gel de silice en éluant avec un mélange 99/1 puis 97/3 de dichlorométhane et de méthanol. On obtient ainsi 0,80 g de produit sous forme d'un solide blanc.

Point de fusion (°C) : 122-124°C

LC-MS : M+H = 253

RMN-¹H (DMSO) δ (ppm) : 9,10 (s, 1H), 8,72 (s, 1H), 8,30 (d, 1H), 8,20 (d, 1H), 7,92 (t, 1H), 7,75 (s, 1H).

### 4.3.{[(3aR,5s,6aS)-2-(5'-fluoro-2,3'-bipyridin-6-yl)octahydrocyclopenta[c]pyrrol-5-yl]méthyl}carbamate de (1-méthyl-1H-1,2,4-triazol-5-yl)méthyle

A une solution de 0,084 g (0,74 mmole) de (2-méthyl-2*H-*[1,2,4]triazol-3-yl)méthanol dans 5 mL de 1,2-dichloroéthane est ajouté 0,26 mL (1,48 mmole) de *N,N-*diisopropyléthylamine, 0,01 g (0.07 mmole) de *N,N-*diméthylaminopyridine et 0,30 g (0,74 mmole) de (3a*R*, 5*s*, 6a*S*) -5- ({[(4-nitrophénoxy)carbonyl]amino}méthyl)hexahydrocyclopenta[*c*]pyr role-2(1*H*)-carboxylate de *tert*-butyle, obtenu à l'étape 4.1.. Le mélange est agité à température ambiante pendant 5 heures.

Après ajout d'eau, on extrait trois fois la phase aqueuse avec du dichlorométhane. Les phases organiques réunies sont successivement lavées par une solution aqueuse de soude 1M puis par une solution aqueuse saturée en chlorure d'ammonium. On sèche la phase organique sur sulfate de sodium, on filtre et on évapore à sec. On obtient ainsi 0,31 g de produit brut engagé tel quel dans l'étape qui suit. 0,28 g (0,74 mmole) de (3a*R*,5*s*,6a*S*)-5-[({[(1-méthyl-1*H-*1,2,4-triazol-5-yl)méthoxy]carbonyl}amino)méthyl]hexahydrocyclopenta[*c*]pyrro le-2(1*H*)-carboxylate de *tert*-butyle, obtenu précédemment, est mis en solution dans 5 mL de dichlorométhane puis 0,62 mL (7,40 mmoles) d'acide trifluoroacétique sont ajoutés. Le mélange est agité à température ambiante pendant 15 heures. Un traitement avec de la soude 1M après extraction au dichlorométhane puis séchage sur sulfate de sodium et évaporation à sec, conduit à 0,18 g d'une huile orange engagée telle quelle dans l'étape suivante, selon le mode opératoire décrit dans l'exemple 1, étape 1.5..

A partir de 0,06 g (0,21 mmole) de [(3a*R*,5*s*,6a*S*)-octahydrocyclopenta[*c*]pyrrol-5-ylméthyl]carbamate de (1-méthyl-1*H*-1,2,4-triazol-5-yl)méthyle, obtenu précédemment, de 0,07 mL (0,43 mmole) de *N,N*-diisopropyléthylamine, de 0,05 g (0,21 mmole) de 6-bromo-5'-fluoro-[2,3']bipyridinyle, obtenu à l'étape 4.2., on obtient 0,022 g de produit sous forme d'un solide blanc.

Point de fusion (°C) : 136-138°C

LC-MS : M+H = 452

RMN-¹H (DMSO) δ (ppm) : 9,15 (s, 1H), 8,60 (s, 1H), 8,30 (m, 1H), 7,90 (s, 1H), 7,62 (m, 1H), 7,45 (large s, 1H), 7,31 (m, 1H), 6,57 (d, 1H), 5,15 (s, 2H), 3,88 (s, 3H), 3,68 (m, 2H), 3,28 (m, 2H), 2,99 (m, 2H), 2,85 (m, 2H), 2,25 (m, 1H), 1,60 (m, 4H).

### Exemple 5 (Composé N°9)

### ({(3aR,5s,6aS)-2-[5-(4-fluorophényl)pyridin-2-yl]octahydrocyclopenta[c]pyrrol-5-yl}méthyl)carbamate de (3-carbamoylisoxazol-5-yl)méthyle (exo)

### 5.1. (3aR,5s,6aS)-5-[({[(3-carbamoylisoxazol-5-yl)méthoxy]carbonyl}amino)méthyl]hexahydrocyclopenta[c]pyrro le-2(1H)-carboxylate de tert-butyle

On procède suivant le mode opératoire décrit dans l'exemple 4, étape 4.3.. A partir de 0,48 g (1,18 mmole) de (3a*R*, 5*s*, 6a*S*) -5- ({[(4-nitrophénoxy)carbonyl]amino}méthyl)hexahydrocyclopenta[*c*]pyr role-2(1*H*)-carboxylate de *tert*-butyle, obtenu dans l'exemple 4, étape 4.1., de 0,17 g (1,18 mmole) de 5-(hydroxyméthyl)-3-isoxazolecarboxamide, 0.41 mL (2.37 mmoles) de *N,N-*diisopropyléthylamine et 0.01 g (0.12 mmole) de N, N-4-diméthylaminopyridine, et après re-cristalllisation dans l'éther diéthylique, on obtient 0,24 g de produit sous forme d'un solide blanc.

Point de fusion (°C) : 186-188°C

LC-MS : M+H = 409

RMN-¹H (DMSO) δ (ppm) : 8,11 (large s, 1H), 7,82 (large s, 1H), 7,48 (large s, 1H), 6,75 (s, 1H), 5,20 (s, 2H), 3,42 (m, 2H), 2,98 (m, 4H), 2,64 (m, 2H), 2,18 (m, 1H), 1,48 (m, 4H), 1,38 (s, 9H).

### 5.2. 2-Fluoro-5-(4-fluoro-phényl)-pyridine

On procède suivant le mode opératoire décrit dans l'exemple 4, étape 4.2. A partir de 2,0 g (14,29 mmoles) d'acide 4-fluoro-benzène boronique, de 5,21 g (14,29 mmoles) de 5-bromo-2-fluoro-pyridine, de 0.82 g (0.71 mmole) de Pd(PPh₃)₄ et de 50 mL d'une solution aqueuse 1M de carbonate de sodium, on obtient 2,30 g de produit sous forme d'un solide blanc.

Point de fusion (°C) : 98-100 °C

LC-MS : M+H = 192

RMN-¹H (DMSO) δ (ppm) : 8,55 (m, 1H), 8,28 (dd, 1H), 7,78 (m, 2H), 7,54 (m, 2H), 7,28 (dd, 1H).

### 5.3. ({(3aR,5s,6aS)-2-[5-(4-fluorophényl)pyridin-2-yl]octahydrocyclopenta[c]pyrrol-5-yl}méthyl) carbamate de (3-carbamoylisoxazol-5-yl)méthyle

On procède suivant le mode opératoire décrit dans l'exemple 4, étape 4.3.. A partir de 0,20 g (1,18 mmole) de (3a*R*,5*s*,6a*S*)-5-[({[(3-carbamoylisoxazol-5-yl)méthoxy]carbonyl}amino)méthyl]hexahydrocyclopenta[*c*]pyrro le-2(1*H*)-carboxylate de *tert*-butyle, obtenu à l'étape 5.1., et de 0,41 mL (4,90 mmoles) d'acide trifluoroacétique, on obtient 0,27 g de [(3a*R*,5*s*,6a*S*)-octahydrocyclopenta[*c*]pyrrol-5-ylméthyl]carbamate de (3-carbamoylisoxazol-5-yl)méthyle sous forme de trifluoroacétate, engagée telle quelle dans l'étape qui suit selon le mode opératoire décrit dans l'exemple 1, étape 1.5..

A partir de 0,10 g (0,24 mmole) de [(3a*R*,5*s*,6a*S*)-octahydrocyclopenta[*c*]pyrrol-5-ylméthyl] carbamate de (3-carbamoylisoxazol-5-yl)méthyle, obtenu précédemment, de 0.16 mL (0.95 mmole) de *N,N*-diisopropyléthylamine et de 0,04 g (0,24 mmole) de 2-fluoro-5-(4-fluoro-phényl)-pyridine, obtenu à l'étape 5.2., et après chromatographie sur plaque préparative de gel de silice en éluant avec un mélange 90/10/1 de dichlorométhane/méthanol/ammoniaque, on obtient 0,018 g de produit sous forme d'un solide blanc.

Point de fusion (°C) : 208-210°C

LC-MS : M+H = 480

RMN-¹H (DMSO) δ (ppm) : 8,40 (s, 1H), 7,95 (large s, 1H), 7,78 (d, 1H), 7,68 (large s, 1H), 7,60 (m, 2H), 7,35 (large s, 1H), 7,22 (m, 2H), 6,75 (s, 1H), 6,52 (d, 1H), 5,19 (s, 2H), 3,61 (m, 2H), 3,22 (m, 2H), 3,01 (m, 2H), 2,82 (m, 2H), 2,25 (m, 1H), 1,60 (m, 4H).

### Exemple 6 (Composé N°10)

### {2-[(3aR,5r,6aS)-2-(5-bromopyridin-2-yl)octahydrocyclopenta[c]pyrrol-5-yl]éthyl} carbamate de [3-(méthylcarbamoyl)isoxazol-5-yl]méthyle (endo)

### 6.1. (3aR,5r,6aS)-5-(2-aminoéthyl)hexahydrocyclopenta[c]pyrrole-2(1H)-carboxylate de tert-butyle

0, 95 g (5,37 mmoles) de diéthyl cyanométhylphosphonate est mis en solution dans 6 mL de tétrahydrofurane puis 0,21 g (5,37 mmoles) d'hydrure de sodium sont ajoutés à température ambiante. Après 10 min d'agitation, 1,1 g (4,88 mmoles) de (3a*R*,6a*S*)-5-oxohexahydrocyclopenta[*c*]pyrrole-2(1*H*)-carboxylate de *tert-*butyle (Tetrahedron, 1993, 49(23), 5047-54) dans 9 mL de tétrahydrofurane sont ajoutées. Le mélange est agité à température ambiante pendant 2 heures.

Après ajout d'eau, le produit est extrait plusieurs fois avec de l'acétate d'éthyle puis les phases organiques réunies sont séchées sur sulfate de sodium. Après filtration et évaporation à sec, on obtient (3a*R*,5*Z*,6a*S*)-5-(cyanométhylidène)hexahydrocyclopenta[*c*]pyrrole-2(1*H*)-carboxylate de *tert*-butyle, engagé tel quel dans l'étape qui suit.

1,24 g (4,88 mmoles) de (3a*R*,5*Z*,6a*S*)-5-(cyanométhylidène)hexahydrocyclopenta[*c*]pyrrole-2(1H)-carboxylate de *tert*-butyle, obtenu précédemment, est mis en solution dans 50 mL d'une solution de soude 1M dans l'éthanol en présence d'une quantité catalytique de Nikel de Raney, à 50% dans l'eau. On agite ensuite, à température ambiante pendant 4 heures, sous une pression d'hydrogène de 4 bars à l'aide d'un appareil de Parr.

Après filtration sur célite et évaporation de l'éthanol, le résidu obtenu est repris dans de l'eau et du dichlorométhane. Le produit est extrait plusieurs fois au dichlorométhane. Les phases organiques réunies sont ensuite lavées à l'aide d'une solution aqueuse saturée en chlorure de sodium puis séchées sur sulfate de sodium. Après filtration et évaporation à sec, on obtient 1,0 g de (3a*R*,5*r*,6a*S*)-5-(2-aminoéthyl)hexahydrocyclopenta[*c*]pyrrole-2(1*H*)-carboxylate de *tert*-butyle, sous forme d'une huile orange utilisée telle quelle dans l'étape suivante.

MS : M⁺ = 254

RMN-¹H (DMSO) δ (ppm) : 3,35 (m, 2H), 3,10 (m, 2H), 2,52 (m, 4H), 2,02 (m, 2H), 1,93 (m, 1H), 1,40 (m, 2H), 1,38 (s, 9H), 0,92 (m, 2H).

### 6.2. (3aR,5r,6aS)-5-{2-[({[3-(éthoxycarbonyl)isoxazol-5-yl]méthoxy}carbonyl)amino]éthyl}hexahydrocyclopenta[c]pyrrol e-2(1H)-carboxylate de tert-butyle

A une solution de 0,33 g (1,97 mmole) de 5-hydroxyméthyl-isoxazole-3-carboxylate d'éthyle et de 0,34 mL (1,97 mmole) de *N,N*-diisopropyléthylamine dans 10 mL de 1,2-dichloroéthane, refroidie à 0°C, on ajoute lentement 0,43 g (2,16 mmoles) de chloroformiate de *p*-nitrophényle en solution dans 5 mL de dichloroéthane. Le mélange est ensuite agité à température ambiante pendant 2 heures puis une solution de 0,5 g (1,97 mmole) de (3a*R*,5*r*,6a*S*)-5-(2-aminoéthyl)hexahydrocyclopenta[*c*]pyrrole-2(1*H*)-carboxylate de *tert*-butyle, obtenu à l'étape 6.1., et 0,34 mL (1,97 mmole) de *N,N*-diisopropyléthylamine sont ajoutés. Le mélange est ensuite chauffé à 70°C pendant 3 heures.

Après retour à température ambiante, de l'eau est ajoutée puis la phase aqueuse est extraite plusieurs fois avec du dichlorométhane. Les phases organiques réunies sont ensuite successivement lavées par une solution aqueuse de soude 1M (trois fois) puis par une solution aqueuse saturée de chlorure d'ammonium (2 fois). On sèche la phase organique sur sulfate de sodium, on filtre et on évapore à sec. Après purification sur colonne de gel de silice en éluant avec un mélange 99/1 puis 97/3 de dichlorométhane et de méthanol, on obtient 0,44 g de produit pur sous forme d'une huile orange.

LC-MS : M+H = 452

RMN-¹H (DMSO) δ (ppm) : 7,40 (large s, 1H), 6,36 (s, 1H), 5,19 (s, 2H), 4,37 (q, 2H), 3,32 (m, 2H), 3,08 (m, 2H), 2,98 (m, 2H), 2,52 (m, 2H), 2,00 (m, 2H), 1,83 (m, 1H), 1,46 (m, 2H), 1,38 (s, 9H), 1,32 (t, 3H), 0,90 (m, 2H).

### 6.3. 5-{[({2-[(3aR,5r,6aS)-2-(5-bromopyridin-2-yl)octahydrocyclopenta[c]pyrrol-5-yl]éthyl}carbamoyl)oxy]méthyl}isoxazole-3-carboxylate d'éthyle

On procède suivant le mode opératoire décrit dans l'exemple 1, étape 1.4. A partir de 0,44 g (0,97 mmole) de (3a*R*,5*r*,6a*S*)-5-{2-[({[3-(éthoxycarbonyl)isoxazol-5-yl]méthoxy}carbonyl)amino]éthyl}hexahydrocyclopenta[*c*]pyrrol e-2(1*H*)-carboxylate de *tert*-butyle, obtenu à l'étape 6.2., de 0,82 mL (9,74 mmoles) d'acide trifluoroacétique, on obtient 5-{[({2-[(3a*R*,5*r*,6a*S*)-octahydrocyclopenta[*c*]pyrrol-5-yl]éthyl}carbamoyl)oxy]méthyl}isoxazole-3-carboxylate d'éthyle sous forme de trifluoroacétate, engagée telle quelle dans l'étape qui suit selon le mode opératoire décrit dans l'exemple 1, étape 1.5..

A partir de 0,45 g (0,97 mmole) de 5-{[({2-[(3a*R*,5*r*,6a*S*)-octahydrocyclopenta[c]pyrrol-5-yl]éthyl}carbamoyl)oxy]méthyl}isoxazole-3-carboxylate d'éthyle, obtenu précédemment, de 0,51 mL (2,91 mmoles) de *N,N*-diisopropyléthylamine, de 0,17 g (0,97 mmole) de 5-bromo-2-fluoro-pyridine, et après chromatographie sur gel de silice en éluant avec un mélange 99/1 à 98/2 de dichlorométhane et de méthanol, on obtient 0,19 g de produit pur sous forme d'un solide blanc.

Point de fusion (°C) : 105-107°C

LC-MS : M+H = 507

RMN-¹H (DMSO) δ (ppm) : 8,10 (d, 1H), 7,61 (d, 1H), 7,45 (large t, 1H), 6,86 (s, 1H), 6,45 (d, 1H), 5,19 (s, 2H), 4,37 (q, 2H), 3,42 (m, 2H), 3,28 (m, 2H), 3,00 (m, 2H), 2,68 (m, 2H), 2,11 (m, 2H), 1,89 (m, 1H), 1,54 (m, 2H), 1,31 (t, 3H), 0,97 (m, 2H).

### 6.4. {2-[(3aR,5r,6aS)-2-(5-bromopyridin-2-yl)octahydrocyclopenta[c]pyrrol-5-yl]éthyl} carbamate de [3-(méthylcarbamoyl)isoxazol-5-yl]méthyle

0, 070 g (0,14 mmole) de 5-{[({2-[(3a*R*,5*r*,6a*S*)-2-(5-bromopyridin-2-yl)octahydrocyclopenta[c]pyrrol-5-yl]éthyl}carbamoyl)oxy]méthyl}isoxazole-3-carboxylate d'éthyle, obtenu à l'étape 6.3., est mis en solution dans 1,7 mL d'une solution 8M de méthylamine dans l'éthanol. Le mélange est agité à température ambiante pendant 2 heures puis évaporer à sec. Après recristallisation à chaud dans l'éther, on obtient 0,031 g de produit sous forme d'un solide blanc.

Point de fusion (°C) : 157-159°C

LC-MS : M+H = 492

RMN-¹H (DMSO) δ (ppm) : 8,67 (m, 1H), 8,10 (d, 1H), 7,60 (dd, 1H), 7,39 (large t, 1H), 6,74 (s, 1H), 6,45 (d, 1H), 5,16 (s, 2H), 3,40 (m, 2H), 3,22 (m, 2H), 3,00 (m, 2H), 2,97 (d, 3H), 2,75 (m, 2H), 2,10 (m, 2H), 1,89 (m, 1H), 1,46 (m, 2H), 0,98 (m, 2H).

### Exemple 7 (Composé N°12)

### {[(3aR,5r,6aS)-5-fluoro-2-(6-fluoroquinolin-2-yl)octahydrocyclopenta[c]pyrrol-5-yl]méthyl} carbamate de (3-carbamoylisoxazol-5-yl)méthyle (exo)

### 7.1. (3aR,5r,6aS)-5-cyano-5-hydroxyhexahydrocyclopenta[c]pyrrole-2(1H)-carboxylate de tert-butyle

1,0 g (4,44 mmoles) de (3a*R*,6a*S*)-5-oxohexahydrocyclopenta[*c*]pyrrole-2(1*H*)-carboxylate de *tert-*butyle (Tetrahedron, 1993, 49(23), 5047-54) sont mis en solution dans un 5 mL d'un mélange eau/méthanol (1/1). Le milieu réactionnel est refroidi à 0°C puis 0,21 g (4,44 mmoles) de cyanure de sodium et 0,47 g (8,44 mmoles) de chlorure d'ammonium sont ajoutés. Le mélange est agité à température ambiante pendant 20 heures.

On évapore à sec puis on reprend le résidu avec du dichlorométhane et de l'eau. On extrait la phase aqueuse plusieurs fois avec du dichlorométhane. On sèche les phases organiques réunies sur sulfate de sodium, on filtre et on évapore à sec. On obtient ainsi 0,59 g de produit sous forme d'un solide blanc, utilisé tel quel dans l'étape suivante.

Point de fusion (°C) : 168-170°C

RMN-¹H (DMSO) δ (ppm) : 6,34 (large s, 1H), 3,40 (m, 2H), 3,20 (m, 2H), 2,82 (m, 2H), 2,28 (m, 2H), 1,82 (m, 2H), 1,40 (s, 9H).

### 7.2. (3aR,5r,6aS)-5-cyano-5-fluorohexahydrocyclopenta[c]pyrrole-2(1H)-carboxylate de tert-butyle

Une solution de 0,50 g (1,98 mmole) de (3a*R*,5*r*,6a*S*)-5-cyano-5-hydroxyhexahydrocyclopenta[*c*]pyrrole-2(1*H*)-carboxylate de *tert*-butyle, obtenu à l'étape 7.1., de 0,32 g (1,98 mmole) de diéthylaminosulfur trifluoride (DAST) dans 6 mL de dichlorométhane est agitée à température ambiante pendant 30 minutes.

On additionne une solution aqueuse saturée d'hydrogénocarbonate de sodium puis on extrait plusieurs fois la phase aqueuse avec du dichlorométhane. On sèche Les phases organiques réunies sur sulfate de sodium, on filtre et on évapore à sec. On obtient ainsi 0,47 g de produit sous forme d'une huile orange utilisé tel quel dans l'étape suivante.

MS : M+H = 255

RMN-¹⁹F (DMSO) δ (ppm) : -142,58

### 7.3. (3aR,5r,6aS)-5-(aminométhyl)-5-fluorohexahydrocyclopenta[c]pyrrole-2(1H)-carboxylate de tert-butyle

A une solution de 0,47 g (1,85 mmole) de (3a*R*,5*r*,6a*S*)-5-cyano-5-fluorohexahydrocyclopenta[*c*]pyrrole-2(1*H*)-carboxylate de *tert*-butyle, obtenu à l'étape 7.2. dans 4 mL de tétrahydrofurane, refroidie à 0°C, on additionne lentement 7,39 mL (7,39 mmoles) d'une solution 1M de borane dans le tétrahydrofurane. Le mélange est agité à 0°C pendant 1 heure puis on additionne lentement de l'éthanol. On poursuit l'agitation pendant environ 3 minutes.

On laisse revenir à température ambiante puis on évapore à sec. Le résidu obtenu est repris par une solution aqueuse saturée de chlorure d'ammonium et du dichlorométhane. On extrait plusieurs fois la phase aqueuse avec du dichlorométhane. On sèche Les phases organiques réunies sur sulfate de sodium, on filtre et on évapore à sec.

On obtient ainsi 0,42 g de produit, utilisé tel quel dans l'étape suivante.

LC-MS : M+H = 259

RMN-¹⁹F (DMSO) δ (ppm) : -143,43

### 7.4. (3aR,5r,6aS)-5-{[({[3-(éthoxycarbonyl)isoxazol-5-yl]méthoxy}carbonyl)amino]méthyl}-5-fluorohexahydrocyclopenta[c]pyrrole-2(1H)-carboxylate de tert-butyle

On procède suivant le mode opératoire décrit dans l'exemple 3, étape 3.4.. A partir de 0,40 g (1,55 mmole) de (3a*R*,5*r*,6a*S*)-5-(aminométhyl)-5-fluorohexahydrocyclopenta[*c*]pyrrole-2(1*H*)-carboxylate de *tert*-butyle, obtenu à l'étape 7.3., de 0,26 g (1,55 mmole) de 5-hydroxyméthyl-isoxazole-3-carboxylate d'éthyle, de 0,54 mL (3,10 mmoles) de *N,N*-diisopropyléthylamine et de 0,31 g (1,55 mmole) de chloroformiate de *para*-nitrophényle, on obtient 0,18 g de produit sous forme d'une huile incolore.

LC-MS: M+H = 455

RMN-¹H (DMSO) δ (ppm) : 7,76 (large t, 1H), 6,90 (s, 1H), 5,25 (s, 2H), 4,38 (q, 2H), 3,38 (m, 2H), 3,30 (m, 2H), 3,18 (m, 2H), 2,70 (m, 2H), 2,05 (m, 2H), 1,74 (m, 2H), 1,40 (s, 9H), 1,33 (t, 3H).

### 7.5. 5-{[({[(3aR,5r,6aS)-5-fluoro-2-(6-fluoroquinolin-2-yl)octahydrocyclopenta[c]pyrrol-5-yl]méthyl}carbamoyl)oxy]méthyl}isoxazole-3-carboxylate d'éthyle

On procède suivant le mode opératoire décrit dans l'exemple 1, étape 1.4.. A partir de 0,17 g (0,37 mmole) de (3a*R*,5*r*,6a*S*)-5-{[({[3-(éthoxycarbonyl)isoxazol-5-yl]méthoxy}carbonyl)amino]méthyl}-5-fluorohexahydrocyclopenta[*c*]pyrrole-2(1*H*)-carboxylate de *tert*-butyle, obtenu à l'étape 7.4., de 0,31 mL (0,425 mmole) d'acide trifluoroacétique, on obtient l'amine 5-{[({[(3a*R*,5*r*,6a*S*)-5-fluorooctahydrocyclopenta[*c*]pyrrol-5-yl]méthyl}carbamoyl)oxy]méthyl}isoxazole-3-carboxylate d'éthyle sous forme de trifluoroacétate, engagée telle quelle dans l'étape qui suit selon le mode opératoire décrit dans l'exemple 1, étape 1.5..

A partir de 0,17 g (0, 37 mmole) de 5-{[({[(3a*R*,5*r*,6a*S*)-5-fluorooctahydrocyclopenta[*c*]pyrrol-5-yl]méthyl}carbamoyl)oxy]méthyl}isoxazole-3-carboxylate d'éthyle, obtenu précédemment, de 0,19 mL (1,11 mmole) de *N,N*-diisopropyléthylamine, de 0,08 g (0,37 mmole) de 2-bromo-6-fluoro-quinoline, et en éluant avec un mélange 99/1 à 97/3 de dichlorométhane et de méthanol, on obtient 0,08 g de produit sous forme d'un solide blanc.

Point de fusion (°C) : 130-132°C

LC-MS : M+H = 501

RMN-¹H (DMSO) δ (ppm) : 8,00 (d, 1H), 7,80 (large t, 1H), 7,59 (m, 1H), 7,50 (dd, 1H), 7,39 (m, 1H), 7,00 (d, 1H), 6,90 (s, 1H), 5,25 (s, 2H), 4,38 (q, 2H), 3,80 (m, 2H), 3,50 (m, 2H), 3,37 (m, 2H), 2,90 (m, 2H), 2,15 (m, 2H), 1,85 (m, 2H), 1,30 (t, 3H).

### 7.6. {[(3aR,5r,6aS)-5-fluoro-2-(6-fluoroquinolin-2-yl)octahydrocyclopenta[c]pyrrol-5-yl]méthyl} carbamate de (3-carbamoylisoxazol-5-yl)méthyle

On procède suivant le mode opératoire décrit dans l'exemple 6, étape 6.4.. A partir de 0,07 g (0,15 mmole) de 5-{[({[(3a*R*,5*r*,6a*S*)-5-fluoro-2-(6-fluoroquinolin-2-yl)octahydrocyclopenta[*c*]pyrrol-5-yl]méthyl}carbamoyl)oxy]méthyl}isoxazole-3-carboxylate d'éthyle, obtenu à l'étape 7.5., et de 2,14 mL d'une solution d'ammoniaque 7M dans le méthanol, et après cristallisation à chaud dans l'éther diéthylique, on obtient 0,04 g de produit sous forme d'un solide blanc.

Point de fusion (°C) : 228-230

LC-MS : M+H = 472

RMN-¹H (DMSO) δ (ppm) : 8,13 (large s, 1H), 8,00 (d, 1H), 7,83 (large s, 1H), 7,77 (large t, 1H), 7,58 (m, 1H), 7,50 (m, 1H), 7,39 (m, 1H), 6,96 (d, 1H), 6,77 (s, 1H), 5,23 (s, 2H), 3,80 (m, 2H), 3,50 (m, 2H), 3,38 (m, 2H), 2,90 (m, 2H), 2,15 (m, 2H), 1,85 (m, 2H).

### Exemple 8 (Composé N°14)

### [3-(6-fluoroquinolin-2-yl)-3-azabicyclo[3.1.0]hex-6-yl]carbamate de [3-(méthylcarbamoyl)isoxazol-5-yl]méthyle

### 8.1 6-[({[3-(éthoxycarbonyl)isoxazol-5-yl]méthoxy}carbonyl)amino]-3-azabicyclo[3.1.0]hexane-3-carboxylate de tert-butyle

On procède suivant le mode opératoire décrit dans l'exemple 3, étape 3.4.. A partir de 3.00 g (15.13 mmole) de 6-amino-3-azabicyclo[3.1.0]hexane-3-carboxylate de *tert*-butyle, de 2.58 g (15.13 mmole) de 5-hydroxyméthyl-isoxazole-3-carboxylate d'éthyle, de 5.27 mL (30.26 mmoles) de *N,N-*diisopropyléthylamine et de 3.05 g (15.13 mmole) de chloroformiate de *para*-nitrophényle, on obtient 4.50 g de produit sous forme d'une huile incolore (75%).

LC-MS: M-H = 394

RMN-¹H (DMSO) δ (ppm): 7.72 (s large, 1H), 6.90 (s, 1H), 5.22 (s, 2H), 4.40 (q, 2H), 3.45 (m, 2H), 3.32 (m, 2H), 2.19 (m, 1H), 1.68 (m, 2H), 1.40 (s, 9H), 1.32 (t, 3H)

### 8.2 5-[({[3-(6-fluoroquinolin-2-yl)-3-azabicyclo[3.1.0]hex-6-yl]carbamoyl}oxy)méthyl]isoxazole-3-carboxylate d'éthyle

On procède suivant le mode opératoire décrit dans l'exemple 1, étape 1.4.. A partir de 4.50 g (11.38 mmole) de 6-[({[3-(éthoxycarbonyl)isoxazol-5-yl]methoxy}carbonyl)amino]-3-azabicyclo[3.1.0]hexane-3-carboxylate de *tert*-butyle, de 9.59 mL (113.81 mmole) d'acide trifluoroacétique, on obtient l'amine 5-{[(3-azabicyclo[3.1.0]hex-6-ylcarbamoyl)oxy]methyl}isoxazole-3-carboxylate d'éthyle sous forme de trifluoroacétate, engagée telle quelle dans l'étape qui suit selon le mode opératoire décrit dans l'exemple 1, étape 1.5..

A partir de 1.55 g (3.79 mmole) de 5-{[(3-azabicyclo[3.1.0]hex-6-ylcarbamoyl)oxy]méthyl}isoxazole-3-carboxylate d'éthyle sous forme de trifluoroacétate, de 1.98 mL (11.37 mmole) de *N,N*-diisopropyléthylamine, de 0.68 g (3.79 mmole) de 2-chloro-6-fluoro-quinoline, et en éluant avec un mélange 99/1 à 98/2 de dichlorométhane et de méthanol, on obtient 0,61 g (36%) de produit sous forme d'une huile orange.

LC-MS : M+H = 441

RMN-¹H (DMSO) δ (ppm) : 8.01 (d, 1H), 7.80 (s large, 1H), 7.64 (m, 1H), 7.50 (m, 1H), 7.40 (m, 1H), 6.95 (m, 2H), 5.25 (s, 2H), 4.38 (q, 2H), 3.85 (m, 2H), 3.55 (m, 2H), 2.35 (m, 1H), 1.90 (m, 2H), 1.30 (m, 3H)

### 8.3 [3-(6-fluoroquinolin-2-yl)-3-azabicyclo[3.1.0]hex-6-yl]carbamate de [3-(méthylcarbamoyl)isoxazol-5-yl] méthyle

On procède suivant le mode opératoire décrit dans l'exemple 6, étape 6.4.. A partir de 0,30 g (0.68 mmole) de 5-[({[3-(6-fluoroquinolin-2-yl)-3-azabicyclo[3.1.0]hex-6-yl]carbamoyl}oxy)méthyl]isoxazole-3-carboxylate d'éthyle et de 8.50 mL d'une solution de méthyle amine 8M dans l'éthanol, et après cristallisation à chaud dans l'éther diéthylique, on obtient 0,17 g (60%) de produit sous forme d'un solide blanc.

Point de fusion (°C) : 220-222°C

LC-MS : M+H = 426

RMN-¹H (DMSO) δ (ppm) : 8,73 (large s, 1H), 8,04 (d, 1H), 7,80 (large s, 1H), 7,62 (m, 1H), 7,54 (m, 1H), 7,42 (m, 1H), 6,97 (d, 1H), 6,81 (s, 1H), 5,22 (m, 2H), 3,86 (m, 2H), 3,57 (m, 2H), 2,80 (d, 3H), 2,35 (m, 1H), 1,90 (m, 2H)

Le tableau 1 qui suit illustre les structures chimiques et les propriétés physiques de quelques composés selon l'invention.

Dans ce tableau, tous les composés sont sous forme de base libre.

**Tableau 1**

| | | | | | | | | | | |
|---|---|---|---|---|---|---|---|---|---|---|
| 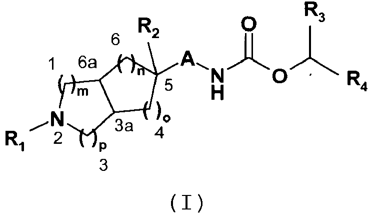 | | | | | | | | | | |

| **N°** | **R₁** | **m** | **n** | **o** | **p** | **A** | **R₂** | **R₃** | **R₄** | **endo ou exo** |
|---|---|---|---|---|---|---|---|---|---|---|
| **1.** | 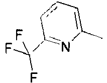 | 1 | 1 | 1 | 1 | CH₂ | H | H | 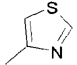 | exo |
| **2.** | 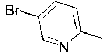 | 1 | 1 | 1 | 1 | CH₂ | H | H | 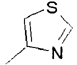 | exo |
| **3.** | 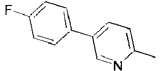 | 1 | 1 | 1 | 1 | CH₂ | H | H | 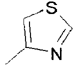 | exo |
| **4.** | 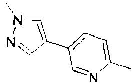 | 1 | 1 | 1 | 1 | CH₂ | H | H | 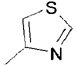 | exo |
| **5.** | 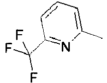 | 1 | 1 | 1 | 1 | liaison | H | H | 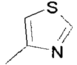 | exo |
| **6.** | 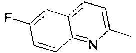 | 1 | 1 | 1 | 1 | liaison | H | H | 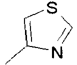 | exo |
| **7.** | 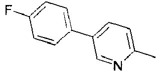 | 1 | 1 | 1 | 1 | CH₂ | H | H | 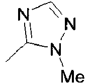 | exo |
| **8.** | 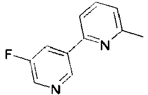 | 1 | 1 | 1 | 1 | CH₂ | H | H | 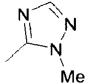 | exo |
| **9.** | 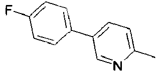 | 1 | 1 | 1 | 1 | CH₂ | H | H | 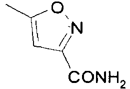 | exo |
| **10.** | 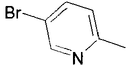 | 1 | 1 | 1 | 1 | (CH₂)₂ | H | H | 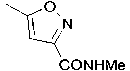 | endo |
| **11.** | 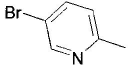 | 1 | 1 | 1 | 1 | (CH₂)₂ | H | H | 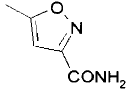 | endo |
| **12.** | 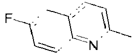 | 1 | 1 | 1 | 1 | CH₂ | F | H | 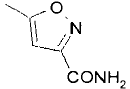 | exo |
| **13.** | 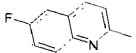 | 1 | 1 | 1 | 1 | CH₂ | H | H | 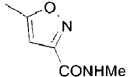 | exo |
| **14.** | 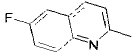 | 1 | 0 | 0 | 1 | liaison | H | H | 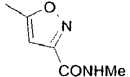 | - |
| **15.** | 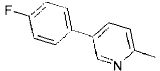 | 1 | 0 | 0 | 1 | liaison | H | H | 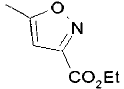 | - |
| **16.** | 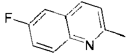 | 1 | 0 | 0 | 1 | liaison | H | H | 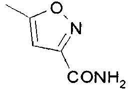 | - |
| **17.** | 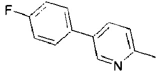 | 1 | 0 | 0 | 1 | liaison | H | H | 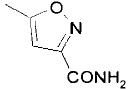 | - |
| **18.** | 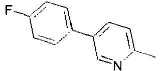 | 1 | 0 | 0 | 1 | liaison | H | H | 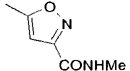 | - |
| **19.** | 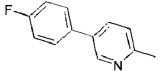 | 1 | 0 | 0 | 1 | liaison | H | H | 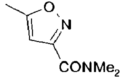 | - |

Le tableau 2 qui suit donne les résultats des analyses RMN ¹H, les points de fusions (PF), et les masses M+H mesurées pour les composés du tableau 1.

**Tableau 2**

| **N°** | ***RMN ¹H (DMSO, 400MHz)*** | **MP** | **M+H** |
|---|---|---|---|
| **1.** | 9,10 (s, 1H), 7,69 (m, 2H), 7,32 (large t, 1H), 7,00 (d, 1H), 6,76 (d, 1H), 5,12 (s, 2H), 3,60 (m, 2H), 3,21 (m, 2H), 3,00 (m, 2H), 2,82 (m, 2H), 2,22 (m, 1H), 1,60 (4H) | 97-99°C | 427 |
| **2.** | 9,10 (s, 1H), 8,12 (s, 1H), 7,65 (m, 2H), 7,30 (large s, 1H), 6,48 (d, 1H), 5,12 (s, 2H), 3,53 (m, 2H), 3,15 (m, 2H), 2,96 (m, 2H), 2,82 (m, 2H), 2,21 (m, 1H), 1,58 (m, 4H) | 114-116°C | 437 |
| **3.** | 9,10 (s, 1H), 8,38 (s, 1H), 7,80 (d, 1H), 7,61 (m, 3H), 7,32 (large s, 1H), 7,21 (m, 2H), 6,58 (d, 1H), 5,11 (s, 2H), 3,60 (m, 2H), 3,12 (m, 2H), 2,99 (m, 2H), 2,81 (m, 2H), 2,21 (m, 1H), 1,56 (m, 4H) | 129-131°C | 453 |
| **4.** | 9,10 (s, 1H), 8,30 (s, 1H), 8,00 (s, 1H), 7,75 (s, 1H), 7,65 (m, 2H), 7,31 (large t, 1H), 6,48 (d, 1H), 5,12 (s, 2H), 3,85 (s, 3H), 3,55 (m, 2H), 3,18 (m, 2H), 3,00 (m, 2H), 2,80 (m, 2H), 2,20 (m, 1H), 1,60 (4H) | 120-122°C | 439 |
| **5.** | 9,11 (s, 1H), 7,70 (m, 2H), 7,40 (m, 1H), 7,01 (d, 1H), 6,78 (d, 1H), 5,12 (s, 2H), 4,00 (m, 1H), 3,58 (m, 2H), 3,28 (m, 2H), 2,90 (m, 2H), 1,78 (m, 4H) | 148-150°C | 413 |
| **6.** | 9,11 (s, 1H), 8,02 (d, 1H), 7,68 (large s, 1H), 7,60 (m, 1H), 7,51 (m, 1H), 7,41 (m, 2H), 7,00 (d, 1H), 5,10 (s, 2H), 4,05 (m, 1H), 3,70 (m, 2H), 3,40 (m, 2H), 2,90 (m, 2H), 1,80 (m, 4H) | 176-178°C | 413 |
| **7.** | 8,40 (s, 1H), 7,91 (s, 1H), 7,80 (d, 1H), 7,62 (m, 2H), 7,45 (large s, 1H), 7,25 (m, 2H), 6,57 (d, 1H), 5,15 (s, 2H), 3,88 (s, 3H), 3,60 (m, 2H), 3,20 (m, 2H), 2,99 (m, 2H), 2, 81 (m, 2H), 2,21 (m, 1H), 1,60 (m, 4H) | 124-126°C | 451 |
| **8.** | 9,15 (s, 1H), 8,60 (s, 1H), 8,30 (m, 1H), 7,90 (s, 1H), 7,62 (m, 1H), 7,45 (large s, 1H), 7,31 (m, 1H), 6,57 (d, 1H), 5,15 (s, 2H), 3,88 (s, 3H), 3,68 (m, 2H), 3,28 (m, 2H), 2,99 (m, 2H), 2,85 (m, 2H), 2,25 (m, 1H), 1,60 (m, 4H) | 136-138°C | 452 |
| **9.** | 8,40 (s, 1H), 7,95 (large s, 1H), 7,78 (d, 1H), 7,68 (large s, 1H), 7,60 (m, 2H), 7,35 (large s, 1H), 7,22 (m, 2H), 6,75 (s, 1H), 6,52 (d, 1H), 5,19 (s, 2H), 3,61 (m, 2H), 3,22 (m, 2H), 3,01 (m, 2H), 2,82 (m, 2H), 2,25 (m, 1H), 1,60 (m, 4H) | 208-210°C | 480 |
| **10** | 8,67 (m, 1H), 8,10 (d, 1H), 7,60 (dd, 1H), 7,39 (large t, 1H), 6,74 (s, 1H), 6,45 (d, 1H), 5,16 (s, 2H), 3,40 (m, 2H), 3,22 (m, 2H), 3,00 (m, 2H), 2,97 (d, 3H), 2,75 (m, 2H), 2,10 (m, 2H), 1,89 (m, 1H), 1,46 (m, 2H), 0,98 (m, 2H) | 157-159°C | 492 |
| **11** | 8.10 (m, 2H), 7.80 (large s, 1H), 7.60 (m, 1H), 7.39 (large t, 1H), 6.73 (s, 1H), 6.45 (d, 1H), 5.16 (s, 2H), 3.41 (m, 2H), 3.26 (m, 2H), 3.00 (m, 2H), 2.68 (m, 2H), 2.09 (m, 2H), 1.89 (m, 1H), 1.47 (m, 2H), 1.00 (m, 2H) | 182-184°C | 478 |
| **12** | 8,13 (large s, 1H), 8,00 (d, 1H), 7,83 (large s, 1H), 7,77 (large t, 1H), 7,58 (m, 1H), 7,50 (m, 1H), 7,39 (m, 1H), 6,96 (d, 1H), 6,77 (s, 1H), 5,23 (s, 2H), 3,80 (m, 2H), 3,50 (m, 2H), 3,38 (m, 2H), 2,90 (m, 2H), 2,15 (m, 2H), 1, 85 (m, 2H) | 228-230°C | 472 |
| **13** | 8,69 (large s, 1H), 8,00 (d, 1H), 7,57 (m, 1H), 7,50 (m, 2H), 7,40 (m, 1H), 6,96 (d, 1H), 6,76 (s, 1H), 5,19 (s, 2H), 3,73 (m, 2H), 3,31 (m, 2H), 2,99 (m, 2H), 2,84 (m, 2H), 2,78 (s, 3H), 2,24 (m, 1H), 1,65 (m, 2H), 1, 56 (m, 2H) | 169-171°C | 468 |
| **14** | 8,73 (large s, 1H), 8,04 (d, 1H), 7,80 (large s, 1H), 7,62 (m, 1H), 7,54 (m, 1H), 7,42 (m, 1H), 6,97 (d, 1H), 6,81 (s, 1H), 5, 22 (m, 2H), 3, 86 (m, 2H), 3, 57 (m, 2H), 2,80 (d, 3H), 2, 35 (m, 1H), 1,90 (m, 2H) | 220-222°C | 426 |
| **15** | 8.40 (s, 1H), 7.82 (m, 2H), 7.64 (m, 2H), 7.25 (m, 2H), 6.94 (s, 1H), 6.55 (d, 1H), 5.24 (s, 2H), 4.40 (q, 2H), 3.74 (m, 2H), 3.45 (m, 2H), 2.34 (m, 1H), 1.88 (m, 2H), 1.35 (t, 3H) | 183-185°C | 467 |
| **16** | 8,12 (large s, 1H), 8,00 (d, 1H), 7,84 (large s, 2H), 7,60 (m, 1H), 7,50 (m, 1H), 7,41 (m, 1H), 6,95 (d, 1H), 6, 80 (s, 1H), 5,20 (m, 2H), 3,85 (m, 2H), 3,57 (m, 2H), 2,35 (m, 1H), 1,88 (m, 2H) | 206-208°C | 412 |
| **17** | 8.40 (s, 1H), 8.15 (large s, 1H), 7.83 (m, 3H), 7.68 (m, 2H), 7.30 (m, 2H), 6.80 (s, 1H), 6.55 (d, 1H), 5.20 (s, 2H), 3.72 (m, 2H), 3. 44 (m, 2H), 2.33 (m, 1H), 1.88 (m, 2H) | 228-230°C | 438 |
| **18** | 8.70 (large s, 1H), 8.40 (s, 1H), 7.80 (m, 2H), 7.61 (m, 2H), 7.25 (m, 2H), 6.80 (s, 1H), 6.55 (d, 1H), 5.20 (s, 2H), 3.73 (m, 2H), 3.42 (m, 2H), 2.78 (s, 3H), 2.33 (m, 1H), 1.87 (m, 2H) | 217-219°C | 452 |
| **19** | 8.38 (s, 1H), 7.81 (m, 2H), 7.62 (m, 2H), 7.28 (m, 2H), 6.74 (s, 1H), 6.55 (d, 1H), 5.22 (s, 2H), 3.72 (m, 2H), 3.42 (m, 2H), 3.10 (s, 3H), 3.01 (s, 3H), 2.31 (m, 1H), 1.88 (m, 2H) | 195-197°C | 466 |

Les composés de l'invention ont fait l'objet d'essais pharmacologiques permettant de déterminer leur effet inhibiteur de l'enzyme FAAH (Fatty Acid Amide Hydrolase).

### Protocole 1

L'activité inhibitrice a été mise en évidence dans un test radioenzymatique basé sur la mesure du produit d'hydrolyse de l'anandamide [éthanolamine 1-³H] par la FAAH *(*Life Sciences (1995), 56, 1999-2005 et Journal of Biochemical and Biophysical Methods (2004), 60(2), 171-177) . Ainsi, les cerveaux de souris (moins le cervelet) sont prélevés et conservés à -80°C. Les homogénats membranaires sont préparés extemporanément par homogénéisation des tissus à l'aide d'un appareil Precellys^{®} dans le tampon de réaction (Tris-HCl 10 mM pH=8, NaCl 150 mM et acide éthylène-diamine-tétraacétique (EDTA) 1 mM). La réaction enzymatique est conduite dans des plaques de filtration Multiscreen 96puits dans un volume final de 70µl. Du tampon de réaction supplémenté avec de l'albumine de sérum bovin sans acides gras (BSA, 1 mg/ml) est utilisé pour la réaction enzymatique, la dilution des composés et de l'anandamide [éthanolamine 1-³H]. Sont ajoutés successivement dans les puits, du tampon de réaction contenant la BSA (43µl/puits), les composés dilués testés à différentes concentrations (7µl/puits contenant 1% de DMSO) et la préparation membranaire (10µl/puits soit 200 µg de tissu par essai). Après 20 minutes de pré-incubation des composés avec l'enzyme à 25°C, la réaction est démarrée par l'addition d'anandamide [éthanolamine 1-³H] (activité spécifique de 15-20 Ci/mmol) diluée avec de l'anandamide froide (10µl/puits, concentration finale de 10 µM, 0,01µCi par essai). Après 20 minutes d'incubation à 25°C, la réaction enzymatique est arrêtée par addition d'une solution de charbon actif 5M préparée dans un tampon NaCl 1,5M et HCl 0,5M (50µl/puits). Le mélange est agité 10 minutes puis la phase aqueuse contenant l'éthanolamine [1-³H] est récupérée par filtration sous-vide et comptée par scintillation liquide.

### Protocole 2

L'activité inhibitrice a été mise en évidence par technique fluorescente dans un test enzymatique basé sur la mesure du produit d'hydrolyse fluorescent de l'arachidonoyl 7-amino 4-méthyl coumarin amide (AAMC) par la FAAH (Analytical Biochemistry (2005), 343:143-151, J. of Biomolecular Screening (2006), 11(5): 519-527 et J. of Neurosciences Methods (2007), 161: 47-54). Ainsi, les cerveaux de souris (moins le cervelet) sont prélevés et conservés à -80°C. Les homogénats de cerveaux sont préparés extemporanément par homogénéisation des tissus à l'aide d'un appareil Precellys® dans le tampon de réaction (Tris-HCl 10 mM pH=8, NaCl 150 mM et acide éthylène-diamine-tétraacétique (EDTA) 1 mM). La réaction enzymatique est conduite dans des plaques 384 puits noires en polystyrène dans un volume final de 50µL. Du tampon de réaction supplémenté avec de l'albumine de sérum bovin sans acides gras (BSA, 1 mg/ml) est utilisé pour la réaction enzymatique, la dilution des composés et la dilution de l'AAMC. Sont ajoutés successivement dans les puits, du tampon de réaction contenant la BSA (25µl/puits), les composés dilués testés à différentes concentrations (5µl/puits contenant 1% de DMSO) et la préparation membranaire (10µl/puits soit 200 µg de tissu par essai). Après 20 minutes de pré-incubation des composés avec l'enzyme à 25°C, la réaction est démarrée par l'addition de 10µL de substrat par puits (AAMC, concentration finale de 10 µM). Après 40 minutes d'incubation à 37°C, l'aminométhyl coumarin (AMC) produit est mesuré par comptage fluorescent (lecteur de plaque Envision).

Dans les conditions du protocole 1, les composés les plus actifs de l'invention présentent des CI₅₀ (concentration inhibant de 50 % l'activité enzymatique contrôle de la FAAH) comprises entre 0,001 et 1 µM. Par exemple les composés n°1, 3, 4, 7, 8, 10 et 12 ont des CI₅₀ respectives de 200 nM, 0,98 nM, 8,5 nM, 170 nM, 1,1 nM et 2,4 nM.

Dans les conditions du protocole 2, les composés les plus actifs de l'invention présentent des CI₅₀ (concentration inhibant de 50 % l'activité enzymatique contrôle de la FAAH) comprises entre 0,001 et 1 µM. Par exemple le composés n°14 a une CI₅₀ respectives de 12 nM.

Il apparaît donc que les composés selon l'invention ont une activité inhibitrice sur l'enzyme FAAH.

L'activité in vivo des composés de l'invention peut être évaluée dans un test d'analgésie.

Ainsi, l'administration intrapéritonéale (i.p.) de PBQ (phénylbenzoquinone, 2 mg/kg dans une solution de chlorure de sodium à 0,9 % contenant 5 % d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale (p.o.) ou par voie intrapéritonéale (i.p.) en suspension dans du Tween 80 à 0,5 %, 60 minutes ou 120 minutes avant l'administration de PBQ. Dans ces conditions, les composés les plus puissants réduisent de 35 à 80 % le nombre d'étirements induits par le PBQ, dans une gamme de doses comprise entre 1 et 30 mg/kg.

L'enzyme FAAH (Chemistry and Physics of Lipids, (2000), 108, 107-121) catalyse l'hydrolyse des dérivés endogènes d'amides et d'esters de différents acides gras tels que la *N*-arachidonoyléthanolamine (anandamide), la *N*-palmitoyl-éthanolamine, la *N*-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.

Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ou tous autres substrats métabolisés par l'enzyme FAAH, sont impliqués. On peut par exemple citer les maladies et les affections suivantes :
la douleur notamment les douleurs aiguës ou chroniques de type neurogène : migraine, douleurs neuropathiques incluant les formes associées au virus de l'herpès et au diabète et à la chimiothérapie, les douleurs aiguës ou chroniques associées aux maladies inflammatoires : arthrite, arthrite rhumatoïde, ostéoarthrite, spondylite, goutte, vascularite, maladie de Crohn, syndrome du colon irritable, les douleurs aiguës ou chroniques périphériques, les vertiges, les vomissements, les nausées en particulier celles consécutives à une chimiothérapie, les troubles du comportement alimentaire en particulier les anorexies et cachexies de diverses natures , les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses, les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires, l'épilepsie, les troubles du sommeil incluant les apnées du sommeil, les maladies cardiovasculaires en particulier hypertension, arythmies cardiaques, artériosclérose, crise cardiaque, ischémies cardiaques, l'ischémie rénale, les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaire, astrocytomes, astroblastomes, épendyomes, oligodendrogliomes, tumeur du plexus, neuroépithéliomes, tumeur de l'épiphyse, épendymoblastomes, méningiomes malins, sarcomatoses, mélanomes malins, schwénnomes), les désordres du système immunitaire, notamment les maladies auto-immunes : psoriasis, lupus érythémateux, maladies du tissu conjonctif ou connectivites, syndrome de Sjögrer's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire, les maladies allergiques : l'hypersensibilité immédiate ou retardée, rhinites ou conjonctivites allergiques, dermatites de contact, les maladies infectieuses parasitaires, virales ou bactériennes : SIDA, méningites, les maladies inflammatoires, notamment les maladies articulaires : arthrite, arthrite rhumatoïde, ostéoarthrite, spondylite, goutte, vascularite, maladie de Crohn, syndrome du colon irritable, l'ostéoporose, les affections oculaires : hypertension oculaire, glaucome, les affections pulmonaires : maladies des voies respiratoires, bronchospasmes, toux, asthme, bronchite chronique, obstruction chronique des voies respiratoires, emphysème, les maladies gastro-intestinales: syndrome du colon irritable, désordres inflammatoires intestinaux, ulcères, diarrhées, l'incontinence urinaire et l'inflammation vésicale.

L'utilisation des composés selon l'invention, à l'état de base, de sel d'addition à un acide, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel d'addition à un acide, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel d'addition à un acide, ou un hydrate, ou un solvat pharmaceutiquement acceptable dudit composé, et éventuellement un ou plusieurs excipients pharmaceutiquement acceptables.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel d'addition à un acide, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intra-trachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

L'invention selon un autre de ses aspects, concerne également une méthode de traitement des pathologies ci-dessus indiquées qui comprend l'administration d'une dose efficace d'un composé selon l'invention, d'un de ses sels d'addition à un acide pharmaceutiquement acceptable, d'un solvat ou d'un hydrate dudit composé.

## Revendications

1. Composé répondant à la formule (I) 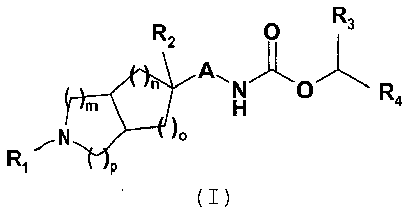 dans laquelle
R₂ représente un atome d'hydrogène, de fluor ou un groupe hydroxyle, cyano, trifluorométhyle, C₁₋₆-alkyle, C₁₋₆-alcoxy ; NR₈R₉ ;
m et p ont pour valeur 1 ;
n et o ont la même valeur et ont pour valeur 0 ou 1 ;
A représente une liaison covalente ou un groupe C₁₋₈-alkylène ;
R₁ représente un groupe R₅ éventuellement substitué par un ou plusieurs groupes R₆ et/ou R₇ ;
R₅ représente un groupe choisi parmi un phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle ;
R₆ représente un atome d'halogène, un groupe cyano,-CH₂CN, nitro, hydroxyle, C₁₋₆-alkyle, C₁₋₆-alcoxy, C₁₋₆-thioalkyle, C₁₋₅-haloalkyle, C₁₋₆-haloalcoxy, C₁₋₆-halothioalkyle, C₃₋₇-cycloalkyle, C₃₋₇-cycloalkyle-C₁₋₃-alkylène, C₃₋₇-cycloalkyle-C₁₋₃-alkylène-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ ou -O-(C₁₋₃-alkylène)-O- ;
R₇ représente un groupe choisi parmi un furanyle, pyrrolyle, thiènyle, oxazolyle, isoxazolyle, thiazolyle, isothiazolyle, imidazolyle, pyrazolyle, phényle, pyridinyle, pyridazinyle, pyrimidinyle, pyrazinyle, triazinyle ; le ou les groupes R₇ pouvant être substitués par un ou plusieurs groupes R₆ identiques ou différents l'un de l'autre ;
R₃ représente un atome d'hydrogène, de fluor, un groupe C₁₋₆-alkyle ou un groupe trifluorométhyle ;
R₄ représente un hétérocycle à 5 chaînons choisi parmi un furanyle, pyrrolyle, thiènyle, thiazolyle, isothiazolyle, oxazolyle, isoxazolyle, pyrrazolyle, oxadiazolyle, thiadiazolyle, imidazole, triazolyle, tétrazolyle;
cet hétérocycle étant éventuellement substitué par un ou plusieurs substituants choisis parmi un atome d'halogène, un groupe C₁₋₆-alkyle, C₁₋₆-haloalkyle, C₃₋₇-cycloalkyle, C₃₋₇-cycloalkyle-C₁₋₃-alkylène, C₁₋₆-haloalcoxy, cyano, NR₈R₉, NR₈COR₉, NR₈CO₂R₉, NR₈SO₂R₉, NR₈SO₂NR₈R₉, COR₈, CO₂R₈, CONR₈R₉, CON (R₈) (C₁₋₃-alkylène-NR₁₀R₁₁), SO₂R₈, SO₂NR₈R₉, -O- (C₁₋₃-alkylène)-O- ;
R₈ et R₉ représentent indépendamment l'un de l'autre un atome d'hydrogène ou un groupe C₁₋₆-alkyle,
ou forment avec le ou les atomes qui les portent,
dans le cas de NR₈R₉, un cycle choisi parmi les cycles azétidine, pyrrolidine, pipéridine, morpholine, thiomorpholine, azépine, oxazépine ou pipérazine, ce cycle étant éventuellement substitué par un groupe C₁₋₆-alkyle ou benzyle ;
dans le cas de NR₈COR₉, un cycle lactame ; dans le cas de NR₈CO₂R₉, un cycle oxazolidinone, oxazinone ou oxazépinone; dans le cas de NR₈SO₂R₉, un cycle sultame ; dans le cas de NR₈SO₂NR₈R₉, un cycle dioxyde de thiazolidine ou dioxyde de thiadiazinane ;
R₁₀ et R₁₁ représentent indépendamment l'un de l'autre un atome d'hydrogène ou un groupe C₁₋₆-alkyle ; à l'état de base ou de sel d'addition à un acide.

2. Composé de formule (I) selon la revendication 1 **caractérisé en ce que** R₂ représente un atome d'hydrogène ou un atome de fluor; à l'état de base ou de sel d'addition à un acide.

3. Composé de formule (I) selon la revendication 1 ou 2 **caractérisé en ce que** m, n, o et p ont pour valeur 1; à l'état de base ou de sel d'addition à un acide.

4. Composé de formule (I) selon l'une quelconque des revendications 1 à 3 **caractérisé en ce que** A représente une liaison covalente, un méthylène ou un éthylène; à l'état de base ou de sel d'addition à un acide.

5. Composé de formule (I) selon l'une quelconque des revendications 1 à 4 **caractérisé en ce que** R₁ représente un groupe R₅ non substitué ou substitué par un ou plusieurs groupes R₆ et/ou R₇ ;
R₅ représente un groupe pyridinyle ou quinolinyle ; R₆ représente un atome d'halogène, plus particulièrement un atome de fluor ou de brome, ou un groupe C₁₋₆-haloalkyle, plus particulièrement trifluorométhyle, ou un groupe C₁₋₆-alkyle, plus particulièrement un groupe méthyle;
R₇ représente un groupe choisi parmi pyrazolyle, pyridinyle et phényle, ces derniers pouvant être substitué par un ou plusieurs groupes R₆ identiques ou différents l'un de l'autre; à l'état de base ou de sel d'addition à un acide.

6. Composé de formule (I) selon l'une quelconque des revendications 1 à 5 **caractérisé en ce que** R₃ représente un atome d'hydrogène; à l'état de base ou de sel d'addition à un acide.

7. Composé de formule (I) selon l'une quelconque des revendications 1 à 6 **caractérisé en ce que** R₄ représente un groupe choisi parmi un thiazolyle, un triazolyle, ou un isoxazolyle; ce groupe étant non substitué ou substitué par un ou plusieurs groupes C₁₋₆-alkyle, CO₂R₈ ou CONR₈R₉; R₈ et R₉ représentent indépendamment l'un de l'autre un atome d'hydrogène ou un groupe C₁₋₆-alkyle ; à l'état de base ou de sel d'addition à un acide.

8. Composé de formule (I) choisi parmi :
({(3a*R*,5*s*,6a*S*)-2-[6-(trifluoromethyl)pyridin-2-yl]octahydrocyclopenta[*c*]pyrrol-5-yl}méthyl)carbamate de thiazol-4-ylméthyle (exo) ;
{[(3a*R*,5*s*,6a*S*)-2-(5-bromopyridin-2-yl)octahydrocyclopenta[*c*]pyrrol-5-yl]méthyl}carbamate de thiazol-4-ylméthyle (exo) ;
({(3a*R*,5*s*,6a*S*)-2-[5-(4-fluorophenyl)pyridin-2-yl]octahydrocyclopenta[*c*]pyrrol-5-yl}méthyl)carbamate de thiazol-4-ylméthyle (exo) ;
({(3a*R*,5*s*,6a*S*)-2-[5-(1-méthyl-1*H*-pyrazol-4-yl)pyridin-2-yl]octahydrocyclopenta[*c*]pyrrol-5-yl}méthyl)carbamate de thiazol-4-ylméthyle (exo) ;
{(3a*R*,5*s*,6a*S*)-2-[6-(trifluorométhyl)pyridin-2-yl]octahydrocyclopenta[*c*]pyrrol-5-yl}carbamate de thiazol-4-ylméthyle (exo) ;
[(3a*R*,5*s*,6a*S*)-2-(6-fluoroquinolin-2-yl)octahydrocyclopenta[*c*]pyrrol-5-yl]carbamate de thiazol-4-ylméthyle (exo) ;
({(3*a*R,5*s*,6a*S*)-2-[5-(4-fluorophenyl)pyridin-2-yl]octahydrocyclopenta[*c*]pyrrol-5-yl}methyl)carbamate de (1-méthyl-1*H*-1,2,4-triazol-5-yl)méthyle (exo) ;
{[(3a*R*,5*s*,6a*S*)-2-(5'-fluoro-2,3'-bipyridin-6-yl)octahydrocyclopenta[*c*]pyrrol-5-yl]méthyl}carbamate de (1-méthyl-1*H*-1,2,4-triazol-5-yl)méthyle (exo)
({(3a*R*,5*s*,6a*S*)-2-[5-(4-fluorophényl)pyridin-2-yl]octahydrocyclopenta[*c*]pyrrol-5-yl}méthyl)carbamate de (3-carbamoylisoxazol-5-yl)méthyle (exo) ;
{2-[(3a*R*,5*r*,6a*S*)-2-(5-bromopyridin-2-yl)octahydrocyclopenta[*c*]pyrrol-5-yl]éthyl}carbamate de [3-(méthylcarbamoyl)isoxazol-5-yl]méthyle (endo) ;
{2-[(3a*R*,5*r*,6a*S*)-2-(5-bromopyridin-2-yl)octahydrocyclopenta[*c*]pyrrol-5-yl]éthyl}carbamate de (3-carbamoylisoxazol-5-yl)méthyle (endo) ;
{[(3a*R*,5*r*,6a*S*)-5-fluoro-2-(6-fluoroquinolin-2-yl)octahydrocyclopenta[*c*]pyrrol-5-yl]méthyl}carbamate de (3-carbamoylisoxazol-5-yl)méthyle (exo) ;
{[(3a*R*,5*s*,6a*S*)-2-(6-fluoroquinolin-2-yl)octahydrocyclopenta[*c*]pyrrol-5-yl]méthyl}carbamate de [3-(méthylcarbamoyl)isoxazol-5-yl]méthyle (exo) ;
[3-(6-fluoroquinolin-2-yl)-3-azabicyclo[3.1.0]hex-6-yl]carbamate de [3-(méthylcarbamoyl)isoxazol-5-yl]méthyle ;
5-{[({3-[5-(4-fluorophényl)pyridin-2-yl]-3-azabicyclo[3.1.0]hex-6-yl}carbamoyl)oxy]méthyl}isoxazole-3-carboxylate d'éthyle ;
[3-(6-fluoroquinolin-2-yl)-3-azabicyclo[3.1.0]hex-6-yl]carbamate de (3-carbamoylisoxazol-5-yl)méthyle ;
{3-[5-(4-fluorophényl)pyridin-2-yl]-3-azabicyclo[3.1.0]hex-6-yl}carbamate de (3-carbamoylisoxazol-5-yl)méthyle ;
{3-[5-(4-fluorophényl)pyridin-2-yl]-3-azabicyclo[3.1.0]hex-6-yl}carbamate de [3-(méthylcarbamoyl)isoxazol-5-yl]méthyle ;
{3-[5-(4-fluorophényl)pyridin-2-yl]-3-azabicyclo[3.1.0]hex-6-yl}carbamate de [3-(diméthylcarbamoyl)isoxazol-5-yl]méthyle.

9. Procédé de préparation d'un composé de formule (I) selon l'une quelconque des revendications 1 à 8, comprenant l'étape consistant à faire réagir une amine de formule générale (II), 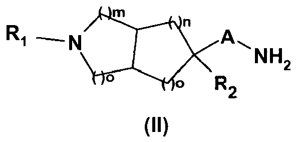 dans laquelle A, R₁, R₂, m, n, o et p sont tels que définis dans la formule générale (I) selon la revendication 1, avec un carbonate de formule générale (III) 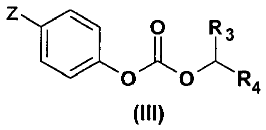 dans laquelle Z représente un atome d'hydrogène ou un groupe nitro, R₃ et R₄ sont tels que définis dans la formule générale (I) selon la revendication 1,
en présence d'une base, dans un solvant à une température comprise entre la température ambiante et la température de reflux du solvant.

10. Procédé de préparation d'un composé de formule (I) selon l'une quelconque des revendications 1 à 8, comprenant l'étape consistant à faire réagir un composé de formule générale (IV) 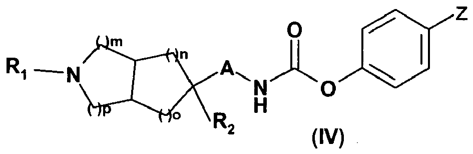 dans laquelle A, R₁, R₂, m, n, o et p sont tels que définis dans la formule générale (I) selon la revendication 1, et Z représente un atome d'hydrogène ou un groupe nitro, avec un alcool de formule générale HOCHR₃R₄ (IIIa) dans laquelle R₃ et R₄ sont tels que définis dans la formule générale (I) selon la revendication 1, en présence d'une base, dans un solvant à une température comprise entre la température ambiante et la température de reflux du solvant.

11. Procédé de préparation d'un composé de formule (I) selon l'une quelconque des revendications 1 à 8, comprenant l'étape consistant à faire réagir un composé de formule générale (Ia) 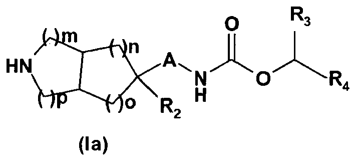 dans laquelle A, R₂, R₃, R₄, m, n, o et p sont tels que définis dans la formule générale (I) selon la revendication 1, avec un dérivé de formule R₁-U₁ (V), dans laquelle R₁ est tel que défini dans la formule générale (I) selon la revendication 1, et U₁ représente un atome d'halogène ou un groupe triflate, en utilisant les conditions de réactions de substitution nucléophiles aromatiques, hétéroaromatiques ou de *N*-arylation, *N*-hétéroarylation de Buchwald.

12. Procédé de préparation d'un composé de formule (I) selon l'une quelconque des revendications 1 à 8, dans laquelle R₁ représente un groupe R₅ substitué notamment par un groupe R₆ de type C₁₋₆-alkyle, C₃₋₇-cycloalkyle ou C₃₋₇-cycloalkyle-C₁₋₃-alkylène, ou par un groupe R₇ tel que défini dans la formule générale (I) selon la revendication 1, comprenant l'étape consistant à réaliser une réaction de couplage, catalysée au moyen d'un métal de transition, sur le composé de formule générale (Ib), 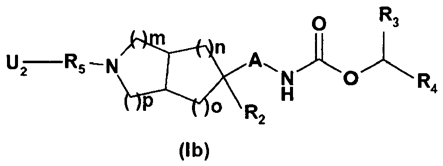 dans laquelle A, R₂, R₃, R₄, R₅, m, n, o et p sont tels que définis dans la formule générale (I) selon la revendication 1 et U₂ représente un atome de chlore, de brome, d'iode ou un groupement triflate, U₂ étant dans la position où l'on souhaite introduire le groupe R₆ ou R₇:
- soit par une réaction de type Suzuki, par exemple au moyen d'un acide boronique d'alkyle, de cycloalkyle, d'aryle ou d'hétéroaryle,
- soit selon une réaction de type Stille, par exemple en utilisant un dérivé *tri*-alkylstanneux d'aryle ou d'hétéroaryle
- soit par une réaction de type Negishi, par exemple en utilisant un dérivé zincate d'halogénure d'alkyle, de cycloalkyle, d'aryle ou d'hétéroaryle.

13. Composé de formule générale (Ia), 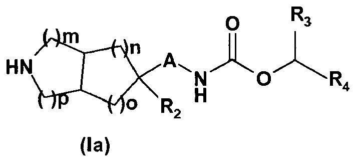 dans laquelle A, R₂, R₃, R₄, m, n, o et p sont tels que définis dans la revendication 1.

14. Composé de formule générale (II), 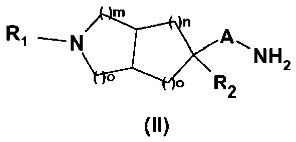 dans laquelle A, R₁, R₂, m, n, o et p sont tels que définis dans la revendication 1.

15. Composé de formule générale (IV), 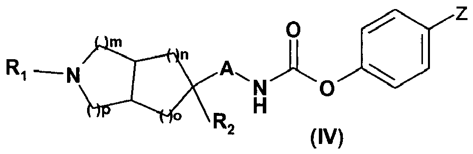 dans laquelle A, R₁, R₂, m, n, o et p sont tels que définis dans la formule générale (I) selon la revendication 1, et Z représente un atome d'hydrogène ou un groupe nitro.

16. Composé de formule (I) selon l'une quelconque des revendications 1 à 8, à l'état de base ou de sel d'addition à un acide pharmaceutiquement acceptable, pour son utilisation comme médicament.

17. Composition pharmaceutique contenant au moins un composé de formule (I) selon l'une quelconque des revendications 1 à 8, à l'état de base ou de sel d'addition à un acide pharmaceutiquement acceptable et éventuellement un ou plusieurs excipients pharmaceutiquement acceptables.

18. Utilisation d'un composé de formule (I) selon l'une quelconque des revendications 1 à 8, à l'état de base ou de sel d'addition à un acide pharmaceutiquement acceptable, pour la préparation d'un médicament destiné à prévenir ou à traiter une pathologie dans laquelle les cannabinoïdes endogènes et/ou tous autres substrats métabolisés par l'enzyme FAAH, sont impliqués.

19. Utilisation d'un composé de formule (I) selon l'une quelconque des revendications 1 à 8, à l'état de base ou de sel d'addition à un acide pharmaceutiquement acceptable, pour la préparation d'un médicament destiné à prévenir ou à traiter les douleurs aiguës ou chroniques de type neurogène, les douleurs aiguës ou chroniques associées aux maladies inflammatoires, les douleurs aiguës ou chroniques périphériques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires, virales ou bactériennes, les maladies inflammatoires, l'ostéoporose, les affections oculaires, les affections pulmonaires, les maladies gastro-intestinales, l'incontinence urinaire ou l'inflammation vésicale.

## Patentansprüche

1. Verbindung der Formel (I) 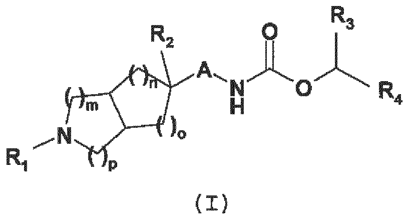 in der
R₂ ein Wasserstoffatom, ein Fluoratom oder eine Hydroxyl-, Cyano-, Trifluormethyl-, C₁₋₆-Alkyl-, C₁₋₆-Alkoxy-, NR₈R₉-Gruppe bedeutet;
m und p einen Wert von 1 haben;
n und o denselben Wert haben und einen Wert von 0 oder
1 haben;
A eine kovalente Bindung oder eine C₁₋₈-Alkylengruppe bedeutet;
R₁ eine R₅-Gruppe, die gegebenenfalls durch eine oder mehrere R₆- und/oder R₇-Gruppen substituiert ist, bedeutet;
R₅ eine Gruppe aus der Reihe Phenyl, Pyridinyl,
Pyridazinyl, Pyrimidinyl, Pyrazinyl, Triazinyl, Naphthalenyl, Chinolinyl, Isochinolinyl, Phthalazinyl, Chinazolinyl, Chinoxalinyl, Cinnolinyl, Naphthyridinyl bedeutet;
R₆ ein Halogenatom, eine Cyano-, -CH₂CN-, Nitro-, Hydroxyl-, C₁₋₆-Alkyl-, C₁₋₆-Alkoxy-, C₁₋₆-Thioalkyl-, C₁₋₆-Halogenalkyl-, C₁₋₆-Halogenalkoxy-, C₁₋₆-Halogenthioalkyl-, C₃₋₇-Cycloalkyl-, C₃₋₇-Cycloalkyl-C₁₋₃-alkylen-, C₃₋₇-Cycloalkyl-C₁₋₃-akylen-O-, NR₈R₉-, NR₈COR₉- , NR₈CO₂R₉-, NR₈SO₂R₉-NR₈SO₂NR₈R₉-, COR₈-, CO₂R₈-, CONR₈R₉-, SO₂R₈-SO₂NR₈R₉- oder -O-(C₁₋₃Alkylen) -O-Gruppe bedeutet; R₇ eine Gruppe aus der Reihe Furanyl, Pyrrolyl, Thienyl, Oxazolyl, Isoxazolyl, Thiazolyl, Isothiazolyl, Imidazolyl, Pyrazolyl, Phenyl, Pyridinyl, Pyridazinyl, Pyrimidinyl, Pyrazinyl, Triazinyl bedeutet; wobei die R₇-Gruppe(n) durch eine oder mehrere R₆-Gruppen, die gleich oder voneinander verschieden sind, substituiert sein können;
R₃ ein Wasserstoffatom, ein Fluoratom, eine C₁₋₆-Alkylgruppe oder eine Trifluormethylgruppe bedeutet;
R₄ einen Heterocyclus mit 5 Kettengliedern, ausgewählt aus Furanyl, Pyrrolyl, Thienyl, Thiazolyl, Isothiazolyl, Oxazolyl, Isoxazolyl, Pyrrazolyl, Oxadiazolyl, Thiadiazolyl, Imidazol, Triazolyl, Tetrazolyl bedeutet;
wobei dieser Heterocyclus gegebenenfalls durch einen oder mehrere Substituenten aus der Reihe Halogenatom, C₁₋₆-Alkyl-, C₁₋₆-Halogenalkyl-, C₃₋₇-Cycloalkyl-, C₃₋₇-Cycloalkyl-C₁₋₃-alkylen-, C₁₋₆-Halogenalkoxy-, Cyano-, NR₈R₉-, NR₈COR₉-, NR₈CO₂R₉-, NR₈SO₂R₉-, NR₈SO₂NR₈R₉-, COR₈-, CO₂R₈-, CONR₈R₉-, CON(R₈) (C₁₋₃-Alkylen-NR₁₀R₁₁)-, SO₂R₈-, SO₂NR₈R₉-, -O-(C₁₋₃-Alkylen) -O-Gruppe substituiert ist;
R₈ und R₉ unabhängig voneinander ein Wasserstoffatom oder eine C₁₋₆-Alkylgruppe bedeuten,
oder mit dem Atom bzw. den Atomen, von dem/denen sie getragen werden,
im Fall von NR₈R₉ einen Zyklus aus der Reihe Azetidin-, Pyrrolidin-, Piperidin-, Morpholin-, Thiomorpholin-, Azepin-, Oxazepin- oder Piperazinzyklen bilden, wobei dieser Zyklus gegebenenfalls durch eine C₁₋₆-Alkyl- oder Benzylgruppe substituiert ist;
im Fall von NR₈COR₉ einen Lactamzyklus bilden; im Fall von NR₈CO₂R₉ einen Oxazolidinon-, Oxazinon- oder Oxazepinonzyklus bilden;
im Fall von NR₈SO₂R₉ einen Sultamzyklus bilden; im Fall von NR₈SO₂NR₈R₉ einen Thiazolidindioxid- oder Thiadiazinandioxidzyklus bilden;
R₁₀ und R₁₁ unabhängig voneinander ein Wasserstoffatom oder eine C₁₋₆-Alkylgruppe bedeuten;
als Base oder als Säureadditionssalz.

2. Verbindung der Formel (I) nach Anspruch 1, **dadurch gekennzeichnet, dass** R₂ ein Wasserstoffatom oder ein Fluoratom bedeutet;
als Base oder als Säureadditionssalz.

3. Verbindung der Formel (I) nach Anspruch 1 oder 2, **dadurch gekennzeichnet, dass** m, n, o und p einen Wert von 1 haben;
als Base oder als Säureadditionssalz.

4. Zusammensetzung der Formel (I) nach einem der Ansprüche 1 bis 3, **dadurch gekennzeichnet, dass** A eine kovalente Bindung, Methylen oder Ethylen bedeutet;
als Base oder als Säureadditionssalz.

5. Zusammensetzung der Formel (I) nach einem der Ansprüche 1 bis 4, **dadurch gekennzeichnet, dass** R₁ eine R₅-Gruppe, die unsubstituiert oder durch eine oder mehrere R₆- und/oder R₇-Gruppen substituiert ist, bedeutet;
R₅ eine Pyridinyl- oder Chinolinylgruppe bedeutet;
R₆ ein Halogenatom, insbesondere ein Fluor- oder Bromatom, oder eine C₁₋₆-Halogenalkylgruppe, insbesondere eine Trifluormethylgruppe, oder eine C₁₋₆-Alkylgruppe, insbesondere eine Methylgruppe, bedeutet; R₇ eine Gruppe aus der Reihe Pyrazolyl, Pyridinyl und Phenyl bedeutet, wobei die letztgenannten durch eine oder mehrere R₆-Gruppen, die gleich oder voneinander verschieden sind, substituiert sein können;
als Base oder als Säureadditionssalz.

6. Verbindung der Formel (I) nach einem der Ansprüche 1 bis 5, **dadurch gekennzeichnet, dass** R₃ ein Wasserstoffatom bedeutet;
als Base oder als Säureadditionssalz.

7. Zusammensetzung der Formel (I) nach einem der Ansprüche 1 bis 6, **dadurch gekennzeichnet, dass** R₄ eine Gruppe aus der Reihe Thiazolyl, Triazolyl oder Isoxazolyl bedeutet, wobei diese Gruppe unsubstituiert oder durch eine oder mehrere C₁₋₆-Alkyl-, CO₂R₈- oder CONR₈R₉-Gruppen substituiert ist; wobei R₈ und R₉ unabhängig voneinander ein Wasserstoffatom oder eine C₁₆-Alkylgruppe bedeuten;
als Base oder als Säureadditionssalz.

8. Verbindung der Formel (I) aus der Reihe:
Thiazol-4-ylmethyl-({(3a*R*,5*s*,6a*S*)-2-[6-(trifluormethyl)pyridin-2-yl]octahydrocyclopenta[*c*]pyrrol-5-yl}methyl)carbamat (exo);
Thiazol-4-ylmethyl-({3a*R*,5*s*,6a*S*)-2-(5-brompyridin-2-yl)octahydrocyclopenta[c]pyrrol-5-yl}methyl)carbamat (exo);
Thiazol-4-ylmethyl-({(3a*R*,5*s*,6a*S*)-2-[5-(4-fluorphenyl)pyridin-2-yl]octahydrocyclopenta[*c*]pyrrol-5-yl}methyl)carbamat (exo);
Thiazol-4-ylmethyl-({(3a*R*,5*s*,6a*S*)-2-[5-(1-methyl-1*H-*pyrazol-4-yl)pyridin-2-yl]octahydrocyclopenta[*c*]pyrrol-5-yl}methyl)carbamat (exo);
Thiazol-4-ylmethyl-[{(3a*R*,5*s*,6a*S*)-2-[6-(trifluormethyl)pyridin-2-yl]octahydrocyclopenta[*c*]pyrrol-5-yl}]carbamät (exo);
Thiazol-4-ylmethyl-[(3a*R*,5*s*,6a*S*)-2-[6-fluorchinolin-2-yl)octahydrocyclopenta[*c*]pyrrol-5-yl]carbamat (exo);
(1-Methyl-1*H*-1,2,4-triazol-5-yl)methyl-({(3a*R*,5*s*,6a*S*)-2-[5-(4-fluorphenyl)pyridin-2-yl]octahydrocyclopenta[*c*]pyrrol-5-yl}methyl)carbamat (exo);
(1-Methyl-1*H*-1,2,4-triazol-5-yl)methyl-({(3a*R*,5*s*,6a*S*)-2-(5'-fluor-2,3'-bipyridin-6-yl)octahydrocyclopenta[*c*]pyrrol-5-yl}methyl)carbamat (exo) ;
(3-Carbamoylisoxazol-5-yl)methyl-({(3a*R*,5*s*,6a*S*)-2-[5-(4-fluorphenyl)pyridin-2-yl]octahydrocyclopenta[*c*]pyrrol-5-yl}methyl)carbamat (exo);
[3-(Methylcarbamoyl)isoxazol-5-yl]methyl-{2-[(3a*R*,5*r*,6a*S*)-2-(5-brompyridin-2-yl)octahydrocyclopenta[*c*]pyrrol-5-yl]ethyl}carbamat (endo);
(3-Carbamoylisoxazol-5-yl)methyl-{2-(3a*R*,5*r*,6a*S*)-2-(5-brompyridin-2-yl)octahydrocyclopenta[*c*]pyrrol-5-yl}ethyl)carbamat (endo);
(3-Carbamoylisoxazol-5-yl)methyl-([(3a*R*,5*r*,6a*S*)-5-fluor-2-(6-fluorchinolin-2-yl)octahydrocyclopenta[*c*]pyrrol-5-yl}methyl)carbamat (exo);
[3-(Methylcarbamoyl)isoxazol-5-yl]methyl-{[(3a*R*,5*s*,6a*S*)-2-(6-fluorchinolin-2-yl)octahydrocyclopenta[*c*]pyrrol-5-yl}methyl)carbamat (exo) ;
[3-(Methylcarbamoyl)isoxazol-5-yl)methyl-[3-(6-fluorchinolin-2-yl)-3-azabicyclo[3.1.0]hex-6-yl]carbamat;
Ethy1-5-{[({3-[5-(4-fluorphenyl)pyridin-2-yl]-3-azabicyclo[3.1.0]hex-6-yl}carbamoyl)oxy]methyl}isoxazol-3-carboxylat;
(3-Carbamoylisoxazol-5-yl)methyl-[3-(6-fluorchinolin-2-yl)-3-azabicyclo[3.1.0]hex-6-yl]carbamat;
(3-Carbamoylisoxazol-5-yl)methyl-{3-[5-(4-fluorphenyl)pyridin-2-yl}-3-azabicyclo[3.1.0]hex-6-yl]carbamat;
[3-(Methylcarbamoyl)isoxazol-5-yl] methyl-{3-[5-(4-fluorphenyl)pyridin-2-yl]-3-azabicyclo[3.1.0]hex-6-yl}carbamat;
[3-(Dimethylcarbamoyl)isoxazol-5-yl]methyl-{3-[5-(4-fluorphenyl)pyridin-2-yl]-3-azabicyclo[3.1.0]hex-6-yl}carbamat;

9. Verfahren zur Herstellung einer Verbindung der Formel (I) nach einem der Ansprüche 1 bis 8, umfassend den Schritt, dass man ein Amin der allgemeinen Formel (II), 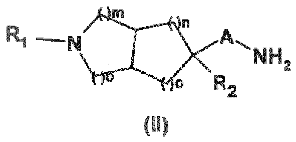 in der A, R₁, R₂, m, n, o und p wie in der allgemeinen Formel (I) nach Anspruch 1 definiert sind, mit einem Carbonat der allgemeinen Formel (III), 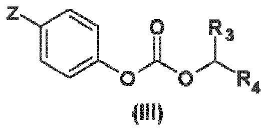 in der Z ein Wasserstoffatom oder eine Nitrogruppe bedeutet, R₃ und R₄ wie in der allgemeinen Formel (I) nach Anspruch 1 definiert sind,
in Gegenwart einer Base in einem Lösungsmittel bei einer Temperatur zwischen Raumtemperatur und der Rückflusstemperatur des Lösungsmittels umsetzt.

10. Verfahren zur Herstellung einer Verbindung der Formel (I) nach einem der Ansprüche 1 bis 8, umfassend den Schritt, dass man eine Verbindung der allgemeinen Formel (IV), 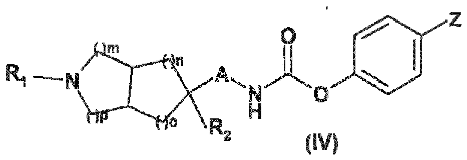 in der A, R₁, R₂, m, n, o und p wie in der allgemeinen Formel (I) nach Anspruch 1 definiert sind und Z ein Wasserstoffatom oder eine Nitrogruppe bedeutet, mit einem Alkohol der allgemeinen Formel HOCHR₃R₄ (IIIa), in der R₃ und R₄ wie in der allgemeinen Formel (I) nach Anspruch 1 definiert sind, in Gegenwart einer Base in einem Lösungsmittel bei einer Temperatur zwischen Raumtemperatur und der Rückflusstemperatur des Lösungsmittels umsetzt.

11. Verfahren zur Herstellung einer Verbindung der Formel (I) nach einem der Ansprüche 1 bis 8, umfassend den Schritt, dass man eine Verbindung der allgemeinen Formel (Ia), 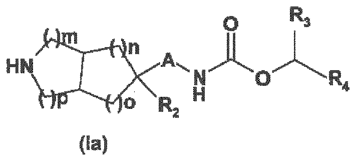 in der A, R₂, R₃, R₄, m, n, o und p wie in der allgemeinen Formel (I) nach Anspruch 1 definiert sind, mit einem Derivat der Formel R₁-U₁ (V), in der R₁ wie in der allgemeinen Formel (I) nach Anspruch 1 definiert ist und U₁ ein Halogenatom oder eine Triflatgruppe bedeutet, unter aromatischen oder heteroaromatischen nukleophilen Substitutionsreaktionsbedingungen oder *N-*Arylierungsbedingungen oder Buchwald-*N-*Heteroarylierungsbedingungen umsetzt.

12. Verfahren zur Herstellung einer Verbindung der Formel (I) nach einem der Ansprüche 1 bis 8, in der R₁ eine R₅-Gruppe bedeutet, die insbesondere durch eine R₆-Gruppe des C₁₋₆-Alkyl-, C₃₋₇-Cycloalkyl- oder C₃₋₇-Cycloalkyl-C₁₋₃-alkylentyps oder durch eine R₇-Gruppe wie in der allgemeinen Formel (I) nach Anspruch 1 definiert substituiert ist, umfassend den Schritt, dass man mit der Verbindung der allgemeinen Formel (Ib), 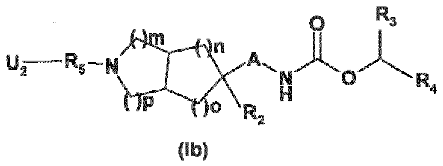 in der A, R₂, R₃, R₄, R₅, m, n, o und p wie in der allgemeinen Formel (I) nach Anspruch 1 definiert sind und U₂ ein Chlor-, Brom-, Iodatom oder eine Triflatgruppierung bedeutet, wobei sich U₂ in der Position befindet, in die man die R₆- oder R₇-Gruppe einzuführen wünscht,
eine Kupplungsreaktion, die von einem Übergangsmetall katalysiert wird, durchführt;
- und zwar entweder mit einer Reaktion des Suzuki-Typs, zum Beispiel mit einer Alkyl-, Cycloalkyl-, Aryl- oder Heteroarylboronsäure,
- oder gemäß einer Reaktion des Stille-Typs, zum Beispiel unter Einsatz eines Aryl- oder Heteroaryltrialkylstannanderivats,
- oder durch eine Reaktion des Negishi-Typs, zum Beispiel unter Einsatz eines Alkyl-, Cycloalkyl-, Aryl- oder Heteroarylhalogenidzinkatderivats.

13. Verbindung der allgemeinen Formel (Ia), 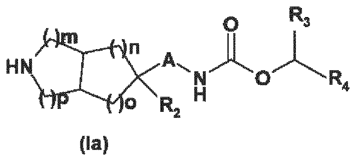 in der A, R₂, R₃, R₄, m, n, o und p wie in Anspruch 1 definiert sind.

14. Verbindung der allgemeinen Formel (II), 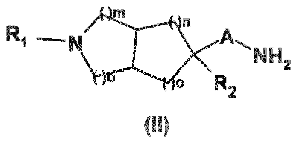 in der A, R₁, R₂, m, n, o und p wie in Anspruch 1 definiert sind.

15. Verbindung der allgemeinen Formel (IV), 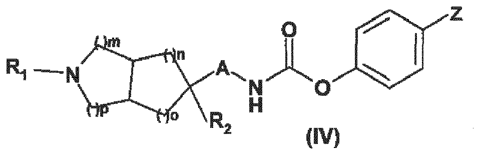 in der A, R₁, R₂, m, n, o und p wie in der allgemeinen Formel (I) nach Anspruch 1 definiert sind und Z ein Wasserstoffatom oder eine Nitrogruppe bedeutet.

16. Verbindung der Formel (I) nach einem der Ansprüche 1 bis 8 als Base oder als Additionssalz mit einer pharmazeutisch unbedenklichen Säure zur Verwendung als Arzneimittel.

17. Pharmazeutische Zusammensetzung, die mindestens eine Verbindung der Formel (I) nach einem der Ansprüche 1 bis 8 als Base oder als Additionssalz mit einer pharmazeutisch unbedenklichen Säure sowie gegebenenfalls einen oder mehrere pharmazeutisch unbedenkliche Träger enthält.

18. Verwendung einer Verbindung der Formel (I) nach einem der Ansprüche 1 bis 8 als Base oder als Additionssalz mit einer pharmazeutisch unbedenklichen Säure zur Herstellung eines Arzneimittels zur Vorbeugung oder Behandlung einer Pathologie, an der endogene Cannabinoide und/oder jegliche sonstige Substrate, die von dem Enzym FAAH metabolisiert werden, impliziert sind.

19. Verwendung einer Verbindung der Formel (I) nach einem der Ansprüche 1 bis 8 als Base oder als Additionssalz mit einer pharmazeutisch unbedenklichen Säure zur Herstellung eines Arzneimittels zur Vorbeugung oder Behandlung von akuten oder chronischen Schmerzen des neurogenen Typs, akuten oder chronischen Schmerzen, die mit Entzündungserkrankungen einhergehen, akuten oder chronischen peripheren Schmerzen, Schwindel, Erbrechen, Übelkeit, Essverhaltensstörungen, neurologischen und psychiatrischen Pathologien, akuten oder chronischen neurodegenerativen Erkrankungen, Epilepsie, Schlafstörungen, Herz-Kreislauf-Erkrankungen, Nierenischämie, Karzinomen, Störungen des Immunsystems, Allergien, parasitären, viralen oder bakteriellen Infektionserkrankungen, entzündlichen Erkrankungen, Osteoporose, Augenleiden, Lungenleiden, Magen-Darm-Krankheiten, Harninkontinenz oder Blasenentzündung.

## Claims

1. Compound corresponding to formula (I): 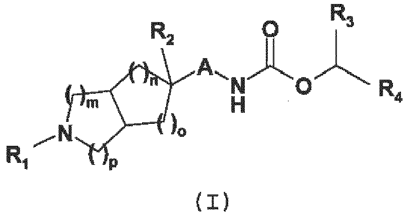 in which
R₂ represents a hydrogen or fluorine atom or a hydroxyl, cyano, trifluoromethyl, C₁₋₆-alkyl, C₁₋₆-alkoxy or NR₈R₉ group;
m and p have the value 1;
n and o have the same value and have the value 0 or 1;
A represents a covalent bond or a group C₁₋₈-alkylene;
R₁ represents a group R₅ optionally substituted with one or more groups R₆ and/or R₇;
R₅ represents a group chosen from phenyl, pyridyl, pyridazinyl, pyrimidinyl, pyrazinyl, triazinyl, naphthalenyl, quinolinyl, isoquinolinyl, phthalazinyl, quinazolinyl, quinoxalinyl, cinnolinyl and naphthyridinyl;
R₆ represents a halogen atom or a cyano, -CH₂CN, nitro, hydroxyl, C₁₋₆-alkyl, C₁₋₆-alkoxy, C₁₋₆-thioalkyl, C₁₋₆-haloalkyl, C₁₋₆-haloalkoxy, C₁₋₆-halothioalkyl, C₃₋₇-cycloalkyl, C₃₋₇-cycloalkyl-C₁₋₃-alkylene, C₃₋₇-cycloalkyl-C₁₋₃-alkylene-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ or -O-(C₁₋₃-alkylene)-O- group;
R₇ represents a group chosen from furyl, pyrrolyl, thienyl, oxazolyl, isoxazolyl, thiazolyl, isothiazolyl, imidazolyl, pyrazolyl, phenyl, pyridyl, pyridazinyl, pyrimidinyl, pyrazinyl and triazinyl; the group(s) R₇ possibly being substituted with one or more groups R₆ that may be identical to or different from each other;
R₃ represents a hydrogen or fluorine atom, a group C₁₋₆-alkyl or a trifluoromethyl group;
R₄ represents a 5-membered heterocycle chosen from furyl, pyrrolyl, thienyl, thiazolyl, isothiazolyl, oxazolyl, isoxazolyl, pyrazolyl, oxadiazolyl, thiadiazolyl, imidazole, triazolyl and tetrazolyl;
this heterocycle being optionally substituted with one or more substituents chosen from a halogen atom, a C₁₋₆-alkyl, C₁₋₆-haloalkyl, C₃₋₇-cycloalkyl, C₃₋₇-cycloalkyl-C₁₋₃-alkylene, C₁₋₆-haloalkoxy, cyano, NR₈R₉, NR₈COR₉, NR₈CO₂R₉, NR₈SO₂R₉, NR₈SO₂NR₈R₉, COR₈, CO₂R₈, CONR₈R₉, CON(R₈)(C₁₋₃-alkylene-NR₁₀R₁₁), SO₂R₈, SO₂NR₈R₉ or -O-(C₁₋₃-alkylene)-O- group;
R₈ and R₉ represent, independently of each other, a hydrogen atom or a C₁₋₆-alkyl group,
or form, with the atom(s) that bear(s) them,
in the case of NR₈R₉, a ring chosen from azetidine, pyrrolidine, piperidine, morpholine, thiomorpholine, azepine, oxazepine and piperazine rings, this ring being optionally substituted with a C₁₋₆-alkyl or benzyl group;
in the case of NR₈COR₉, a lactam ring; in the case of NR₈CO₂R₉, an oxazolidinone, oxazinone or oxazepinone ring; in the case of NR₈SO₂R₉, a sultam ring; in the case of NR₈SO₂NR₈R₉, a thiazolidine dioxide or thiadiazinane dioxide ring;
R₁₀ and R₁₁ represent, independently of each other, a hydrogen atom or a C₁₋₆-alkyl group; in the form of base or of acid-addition salt.

2. Compound of formula (I) according to Claim 1, **characterized in that** R₂ represents a hydrogen atom or a fluorine atom; in the form of base or of acid-addition salt.

3. Compound of formula (I) according to Claim 1 or 2 **characterized in that** m, n, o and p have the value 1; in the form of base or of acid-addition salt.

4. Compound of formula (I) according to any one of Claims 1 to 3, **characterized in that** A represents a covalent bond, a methylene or an ethylene; in the form of base or of acid-addition salt.

5. Compound of formula (I) according to any one of Claims 1 to 4, **characterized in that** R₁ represents a group R₅ which is unsubstituted or is substituted with one or more groups R₆ and/or R₇;
R₅ represents a pyridyl or quinolinyl group;
R₆ represents a halogen atom, more particularly a fluorine or bromine atom, or a C₁₋₆-haloalkyl group, more particularly a trifluoromethyl group or a C₁₋₆-alkyl group, more particularly a methyl group;
R⁷ represents a group chosen from pyrazolyl, pyridyl and phenyl, the latter groups possibly being substituted with one or more groups R₆ that may be identical to or different from each other; in the form of base or of acid-addition salt.

6. Compound of formula (I) according to any one of Claims 1 to 5, **characterized in that** R₃ represents a hydrogen atom; in the form of base or of acid-addition salt.

7. Compound of formula (I) according to any one of Claims 1 to 6, **characterized in that** R₄ represents a group chosen from a thiazolyl, a triazolyl or an isoxazolyl; this group being unsubstituted or substituted with one or more C₁₋₆-alkyl, CO₂R₈ or CONR₈R₉ groups; R₈ and R₉ represent, independently of each other, a hydrogen atom or a C₁₋₆-alkyl group; in the form of base or of acid-addition salt.

8. Compound of formula (I) chosen from:
thiazol-4-ylmethyl ({(3a*R*,5*s*,6a*S*}-2-[6-(trifluoromethyl)pyrid-2-yl]octahydrocyclopenta[*c*]pyrrol-5-yl}methyl)carbamate (exo);
thiazol-4-ylmethyl {[(3a*R*,5*s*,6a*S*)-2-(5-bromopyrid-2-yl)octahydrocyclopenta[*c*]pyrrol-5-yl]methyl}carbamate (exo);
thiazol-4-ylmethyl ({(3a*R*,5*s*,6a*S*)-2-[5-(4-fluorophenyl)pyrid-2-yl]octahydrocyclopenta[*c*]pyrrol-5-yl}methyl)carbamate (exo);
thiazol-4-ylmethyl ({(3a*R*,5*s*,6a*S*)-2-[5-(1-methyl-1*H-*pyrazol-4-yl)pyrid-2-yl]octahydrocyclopenta[*c*]pyrrol-5-yl}methyl)carbamate (exo);
thiazol-4-ylmethyl {(3a*R*,5*s*,6a*S*)-2-[6-(trifluoromethyl)pyrid-2-yl]-octahydrocyclopenta[*c*]pyrrol-5-yl}carbamate (exo);
thiazol-4-ylmethyl [(3a*R*,5*s*,6a*S*)-2-(6-fluoroquinolin-2-yl)octahydrocyclopenta[*c*]pyrrol-5-yl]carbamate (exo);
(1-methyl-1*H*-1,2,4-triazol-5-yl)methyl ({(3a*R*,5*s*,6a*s*)-2-[5-(4-fluorophenyl)pyrid-2-yl]octahydrocyclopenta[*c*]pyrrol-5-yl}methyl)carbamate (exo) ;
(1-methyl-1*H*-1,2,4-triazol-5-yl}methyl {[(3a*R*,5*s*,6a*S*)-2-(5'-fluoro-2,3'-bipyrid-6-yl)octahydrocyclopenta[*c*]pyrrol-5-yl]methyl}carbamate (exo) ;
(3-carbamoylisoxazol-5-yl)methyl ({(3a*R*,5*s*,6a*S*)-2-[5-(4-fluorophenyl)pyrid-2-yl]octahydrocyclopenta[*c*]pyrrol-5-yl}methyl)carbamate (exo);
[3-(methylcarbamoyl)isoxazol-5-yl]methyl {2-[(3a*R*,5*r*,6a*S*)-2-(5-bromopyrid-2-yl)octahydrocyclopenta[*c*]pyrrol-5-yl]ethyl}carbamate (endo);
(3-carbamoylisoxazol-5-yl)methyl {2-[(3a*R*,5*r*,6a*S*)-2-(5-bromopyrid-2-yl)octahydrocyclopenta[*c*]pyrrol-5-yl] ethyl}carbamate (endo) ;
(3-carbamoylisoxazol-5-yl)methyl {[(3a*R,*5*r*,6a*S*)-5-fluoro-2-{6-fluoroquinolin-2-yl}octahydrocyclopenta[*c*]pyrrol-5-yl]methyl}carbamate (exo);
[3-(methylcarbamoyl)isoxazol-5-yl]methyl ([(3a*R*,5*s*,6a*S*)-2-(6-fluoroquinolin-2-yl)octahydrocyclopenta[*c*]pyrrol-5-yl]methyl}carbamate (exo) ;
[3-(methylcarbamoyl)isoxazol-5-yl]methyl[3-(6-fluoro-quinolin-2-yl)-3-azabicyclo[3.1.0]hex-6-yl]carbamate;
ethyl 5-{[({3-[5-(4-fluorophenyl)pyrid-2-yl]-3-azabicyclo[3.1.0]hex-6-yl}carbamoyl)oxy]methyl}isoxazole-3-carboxylate;
(3-carbamoylisoxazol-5-yl)methyl [3-(6-fluoroquinolin-2-yl)-3-azabicyclo[3.1.0]hex-6-yl]carbamate;
(3-carbamoylisoxazol-5-yl)methyl {3-[5-(4-fluoro-phenyl)pyrid-2-yl]-3-azabicyclo[3.1.0]hex-6-yl}carbamate;
[3-(methylcarbamoyl)isoxazol-5-yl]methyl {3-[5-(4-fluorophenyl)pyrid-2-yl]-3-azabicyclo[3.1.0]hex-6-yl}carbamate;
[3-(dimethylcarbamoyl)isoxazol-5-yl]methyl {3-[5-(4-fluorophenyl)pyrid-2-yl]-3-azabicyclo[3.1.0]hex-6-yl}-carbamate.

9. Process for preparing a compound of formula (I) according to any one of Claims 1 to 8, comprising the step that consists in reacting an amine of general formula (II) : 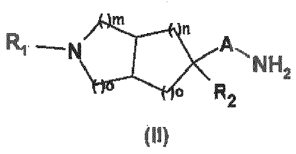 in which A, R₁, R₂, m, n, o and p are as defined in the general formula (I) according to Claim 1, with a carbonate of general formula (III): 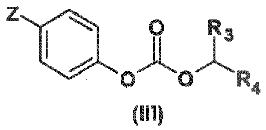 in which Z represents a hydrogen atom or a nitro group, and R₃ and R₄ are as defined in the general formula (I) according to Claim 1,
in the presence of a base, in a solvent at a temperature between room temperature and the reflux temperature of the solvent.

10. Process for preparing a compound of formula (I) according to any one of Claims 1 to 8, comprising the step that consists in reacting a compound of general formula (IV): 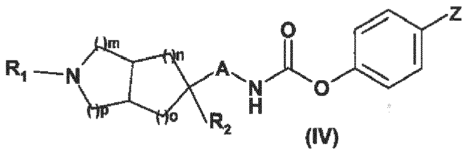 in which A, R₁, R₂, m, n, o and p are as defined in the general formula (I) according to Claim 1, and Z represents a hydrogen atom or a nitro group, with an alcohol of general formula HOCHR₃R₄ (IIIa), in which R₃ and R₄ are as defined in the general formula (I) according to Claim 1, in the presence of a base, in a solvent at a temperature between room temperature and the reflux temperature of the solvent.

11. Process for preparing a compound of formula (I) according to any one of Claims 1 to 8, comprising the step that consists in reacting a compound of general formula (Ia): 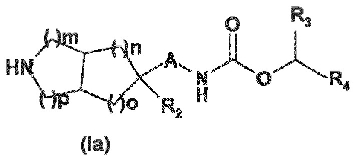 in which A, R₂, R₃, R₄, m, n, o and p are as defined in the general formula (I) according to Claim 1, with a derivative of formula R₁-U₁ (V), in which R₁ is as defined in the general formula (I) according to Claim 1 and U₁ represents a halogen atom or a triflate group, using aromatic or heteroaromatic nucleophilic substitution reaction conditions or using Buchwald N-arylation or *N*-heteroarylation conditions.

12. Process for preparing a compound of formula (I) according to any one of Claims 1 to 8, in which R₁ represents a group R₅ substituted especially with a group R₆ of the C₁₋₆-alkyl, C₃₋₇-cycloalkyl or C₃₋₇-cycloalkyl-C₁₋₃-alkylene type, or with a group R₇ as defined in the general formula (I) according to Claim 1, comprising the step that consists in performing a coupling reaction, catalysed by means of a transition metal, on the compound of general formula (Ib): 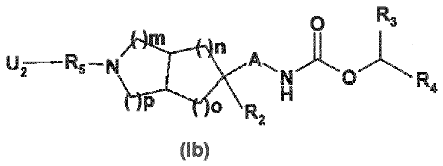 in which A, R₂, R₃, R₄, R₅, m, n, o and p are as defined in the general formula (I) according to Claim 1 and U₂ represents a chlorine, bromine or iodine atom or a triflate group, U₂ being in the position in which it is desired to introduce the group R₆ or R₇:
- either via a reaction of Suzuki type, for example using an alkyl, cycloalkyl, aryl or heteroaryl boronic acid ;
- or according to a reaction of Stille type, for example using an aryl or heteroaryl trialkylstannous derivative;
- or via a reaction of Negishi type, for example using an alkyl, cycloalkyl, aryl or heteroaryl halide zincate derivative.

13. Compound of general formula (Ia) : 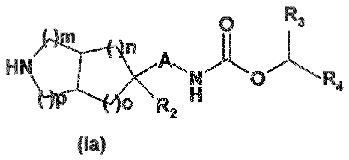 in which A, R₂, R₃, R₄, m, n, o and p are as defined in Claim 1.

14. Compound of general formula (II) : 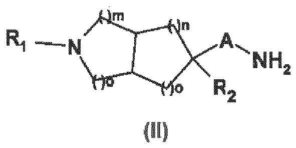 in which A, R₁, R₂, m, n, o and p are as defined in Claim 1.

15. Compound of general formula (IV): 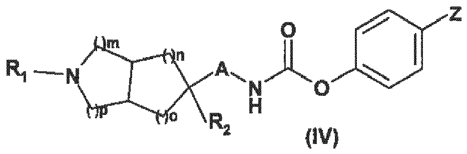 in which A, R₁, R₂, m, n, o and p are as defined in the general formula (I) according to Claim 1, and Z represents a hydrogen atom or a nitro group.

16. Compound of formula (I) according to any one of Claims 1 to 8, in the form of base or of a pharmaceutically acceptable acid-addition salt, for its use as a medicament.

17. Pharmaceutical composition containing at least one compound of formula (I) according to any one of Claims 1 to 8, in the form of base or of a pharmaceutically acceptable acid-addition salt and optionally one or more pharmaceutically acceptable excipients.

18. Use of a compound of formula (I) according to any one of Claims 1 to 8, in the form of base or of a pharmaceutically acceptable acid-addition salt, for the preparation of a medicament for preventing or treating a pathology in which the endogenous cannabinoids and/or any other substrate metabolized by the enzyme FAAH are involved.

19. Use of a compound of formula (I) according to any one of Claims 1 to 8, in the form of base or of a pharmaceutically acceptable acid-addition salt, for the preparation of a medicament for preventing or treating acute or chronic pain of neurogenic type, acute or chronic pain associated with inflammatory diseases, acute or chronic peripheral pain, vertigo, vomiting, nausea, eating disorders, neurological and psychiatric pathologies, acute or chronic neurodegenerative diseases, epilepsy, sleep disorders, cardiovascular diseases, renal ischaemia, cancers, immune system disorders, allergic diseases, parasitic, viral or bacterial infectious diseases, inflammatory diseases, osteoporosis, ocular complaints, pulmonary complaints, gastrointestinal diseases, urinary incontinence or inflammation of the bladder.
